(19) Europäisches Patentamt
European Patent Office
Office européen des brevets

(11) Publication number: 0 300 404 A2

# EUROPEAN PATENT APPLICATION

(21) Application number: 88111507.5

(22) Date of filing: 18.07.88

(51) Int. Cl.⁴: C07C 62/38, C07C 62/34, C07C 62/32, C07C 69/757, C07C 47/47

Claims for the following Contracting State: GR.

(30) Priority: 20.07.87 US 75490

(43) Date of publication of application: 25.01.89 Bulletin 89/04

(84) Designated Contracting States: AT BE CH DE ES FR GB GR IT LI NL SE

(71) Applicant: G.D. Searle & Co.
P.O. Box 5110
Chicago Illinois 60680(US)

(72) Inventor: Miyano, Masateru
2139 Valley Road
Northbrook, IL 60062(US)
Inventor: Stealey, Michael Allan
502 Juniper Parkway
Libertyville, IL 60048(US)
Inventor: Shone, Robert Larry
1441 Joan Drive
Palatine, IL 60067(US)

(74) Representative: Beil, Hans Chr., Dr. et al
Beil, Wolff und Beil, Rechtsanwälte
Adelonstrasse 58 Postfach 80 01 40
D-6230 Frankfurt am Main 80(DE)

## (54) Di-and tetrahydronapthyl anti-allergy agents.

(57) This invention relates to compounds of the formula:

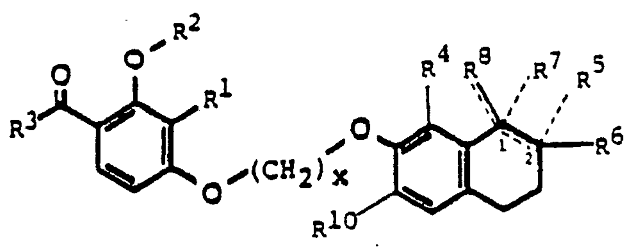

and the pharmaceutically acceptable salts thereof:
wherein $R^1$ represents straight or branched chain alkyl having 2-4 carbon atoms;
wherein $R^2$ represents hydrogen or straight or branched chain alkyl having 1-6 carbon atoms;
wherein $R^3$ represents hydrogen or straight or branched chain alkyl having 1-3 carbon atoms;
wherein $R^4$ and $R^{10}$ are different and represent H or n-propyl;
wherein X represents an interger from 2 to 5;
wherein $R^5$ is hydrogen, or acts with $R^7$ to produce an additional carbon-carbon bond between $C_1$ and $C_2$;
wherein $R^6$ represents hydrogen or $-Z-COOR^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;

EP 0 300 404 A2

wherein $R^7$ is hydrogen, or acts together with $R^5$ to form an additional carbon-carbon bond between $C_1$ and $C_2$, or acts together with $R^8$ to form

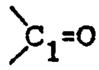

when $R^8$ is oxygen;
wherein $R^8$ is hydrogen, $-O-R^{11}$, or oxygen, such that when $R^8$ is oxygen, $R^8$ acts together with $R^7$ to form

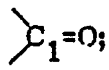

and
wherein $R^{11}$ is straight or branched chain alkyl having 1-4 carbon atoms.

The di- and tetrahydronaphthyl compounds of this invention are pharmacologically active as leukotriene $D_4$ ($LTD_4$) antagonists and are useful as anti-inflammatory and anti-allergy agents.

# DI- AND TETRAHYDRONAPHTHYL ANTI-ALLERGY AGENTS

## BACKGROUND OF THE INVENTION

### (a) Field of the Invention.

This invention relates to novel compounds of Formula I having an all carbon ring-structure which are antagonists of leukotriene $D_4$ ($LTD_4$) and the slow reacting substance of anaphylaxis (SRS-A). In particular, the compounds of this invention are useful as pharmaceutical agents to prevent or alleviate the symptoms associated with $LTD_4$, such as allergic reactions and inflammatory conditions.

$LTD_4$ is a product of the 5-lipoxygenase pathway and is the major active constitutent of slow reacting substance of anaphylaxis (SRS-A), a potent bronchoconstrictor that is released during allergic reactions. See R. A. Lewis and K. F. Austen, Nature, 293, 103-108 (1961). When administered to humans and guinea pigs, $LTD_4$ causes bronchoconstriction by two mechanisms: (1) directly, by stimulating smooth muscle; and (2) indirectly, through release of thromboxane $A_2$ which then causes contraction of respiratory smooth muscle. Because antihistamines are ineffective in the management of asthma, SRS-A is believed to be a mediator in the bronchoconstriction occurring during an allergic attack. $LTD_4$ may also be involved in other inflammatory conditions such as rheumatoid arthritis. Furthermore, $LTD_4$ is a potent coronary vasoconstrictor and influences contractile force in the myocardium and coronary flow rate of the isolated heart. See F. Michelassi, et al., Science, 217, 841 (1982); J. A. Burke, et al. J. Pharmacol. and Exp. Therap., 221, 235 (1982).

### (b) Prior Art.

Appleton et. al, J. Med. Chem. 20, 371-379 (1977) discloses a series of chromone-2-carboxylic acids which are antagonists of SRS-A. Specifically sodium 7-[3-(4-acetyl-3-hydroxy-2-propylphenoxy)-2-hydroxypropoxy]-4-oxo-8-propyl-4H-1-benzopyran-2-carboxylate (FPL 55712), appears to be the first reported specific antagonist of SRS-A and $LTD_4$.

Miyano et. al. (U.S. Pat. 4,546,194) discloses substituted chromanon-2-yl alkanols and derivatives thereof which are useful as $LTD_4$ inhibitors.

Similar references disclosing chromane compounds which are useful as $LTD_4$ antagonists are the following: European Patent Application Nos. 0079637, 129,906, and 150,447; U.S. Pat. 4,595,882; Japanese Patent 60/42378; C.A. 103(19) 160 389 G; and J. Medicinal Chem, 20 (3) 376 (1977).

## SUMMARY OF THE INVENTION

This invention encompasses compounds of the formula

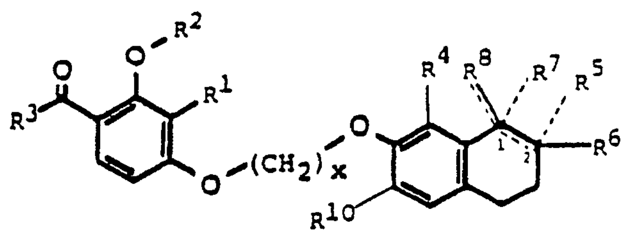

I

and the pharmaceutically acceptable salts thereof;
wherein $R^1$ represents straight or branched chain alkyl having 2-4 carbon atoms;

wherein $R^2$ represents hydrogen or straight or branched chain alkyl having 1-6 carbon atoms; wherein $R^3$ represents hydrogen or straight or branched chain alkyl having 1-3 carbon atoms; wherein $R^4$ and $R^{10}$ are different and represent hydrogen or n-propyl; wherein $R^5$ is hydrogen, or acts with $R^7$ to produce an additional carbon-carbon bond between $C_1$ and $C_2$; wherein X represents an integer from 2 to 5; wherein $R^6$ represents hydrogen or $-Z-COOR^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms; wherein $R^7$ is hydrogen, or acts together with $R^5$ to form an additional carbon-carbon bond between $C_1$ and $C_2$, or acts together with $R^8$ to form

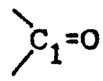

when $R^8$ is oxygen;
wherein $R^8$ is hydrogen, $-O-R^{11}$, or oxygen, such that when $R^8$ is oxygen, $R^8$ acts together with $R^7$ to form

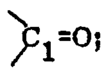

and
wherein $R^{11}$ is straight or branched chain alkyl having 1-4 carbon atoms.

It is the object of the present invention to produce $LTD_4$ antagonists which do not require the oxygen containing chromane ring structure. More specifically, it is the object of this invention to produce compounds, which contain an all carbon ring structure, and have $LTD_4$ antagonistic activity.

## DETAILED DESCRIPTION

This invention encompasses compounds of Formula I as previously described. A preferred embodiment of this invention are compounds of the formula:

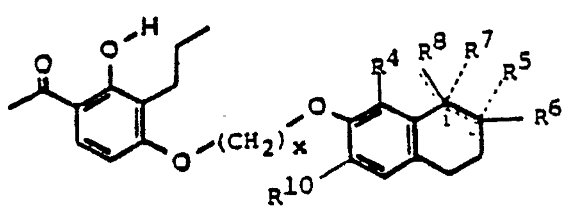

II

and the pharmaceutically acceptable salts thereof;
wherein X, $R^4$, $R^5$, $R^6$, $R^7$, $R^8$, $R^9$, Z, $R^{10}$ and $R^{11}$ are as previously described.

A more preferred embodiment of this invention are compounds of the formula:

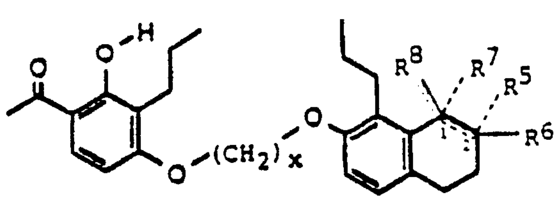

III

and the pharmaceutically acceptable salts thereof;
wherein X, $R^5$, $R^6$, $R^7$, $R^8$, $R^9$, Z, and $R^{11}$ are as previously described.

The term "pharmaceutically acceptable cations" are used to describe $R^9$ refers to cations such as ammonium, sodium, potassium, lithium, calcium, magnesium, ferrous, zinc, copper, manganous, aluminum, ferric, manganic, ammonium, tetraalkylammonium, and the like.

The term "pharmaceutically acceptable addition salts" refers to those base derived salts of any compound herein having a carboxylic acid function.

Among the organic bases employed to produce said pharmaceutically acceptable salts are the pharmaceutically acceptable non-toxic bases of primary, secondary, and tertiary amines. Especially preferred non-toxic bases are isopropylamine, diethylamine, ethanolamine, dicyclohexylamine, choline, and caffeine.

All the pharmaceutically acceptable non-toxic addition salts are prepared by conventional processes well known to those of ordinary skill in the art.

$LTD_4$ acts by causing brochoconstriction in both guinea pigs and humans. The bronchoconstriction has two components: (1) a direct component, wherein $LTD_4$ stimulates the respiratory smooth muscle to constrict; and (2) an indirect component wherein $LTD_4$ causes the release of thromboxane A2 which also causes the constriction of respiratory smooth muscle. The compounds of this invention act by antagonizing the direct constriction of respiratory smooth muscle by $LTD_4$.

The $LTD_4$ antagonistic activity of the compounds of this invention was determined by in vivo testing upon guinea pigs. Specifically, adult male fasted Hartly guinea pigs weighing 300-360g were pretreated with pyrilamine and indomethacin to block the bronchoconstricture effects of endogenous histamine and the synthesis of thromboxane A2 respectively. Compounds of the invention were administered IG (intragastrically) at appropriate times prior to the IV (intravenous) administration of 2000 units of $LTD_4$. Intratracheal pressure was monitored prior to and subsequent to $LTD_4$ administration in animals anesthetized with pentobarbital and attached to a rodent respirator. A compound was determined to antagonize the direct component of $LTD_4$ action on respiratory smooth muscle if the compound inhibited the intratracheal insufflation pressure increases (P or = 0.05) caused by $LTD_4$. The compounds of this invention were found to exhibit $LTD_4$ antagonistic activity at doses of 10mg/Kg.

By virtue of their activity as $LTD_4$ inhibitors, the compounds of Formula I are useful in treating inflammatory conditions in mammals in which $LTD_4$ plays a role such as psoriasis, Crohn's disease, and ulcerative colitis and the like. A physician or veterinarian of ordinary skill can readily determine whether a subject exhibits the inflammatory condition. The preferred utility relates to treatment of ulcerative colitis.

The compounds can be administered in such oral dosage forms as tablets, capsules, softgels, pills, powders, granules, elixirs, or syrups. The compounds may also be administered intravascularly, intraperitoneally, subcutaneously, intramuscularly, or topically using forms known to the pharmaceutical art. In general, the preferred form of administration is oral or in such a manner so as to localize the inhibitor. For example, for asthma, the compounds could be inhaled using an aerosol or other appropriate spray. In an inflammatory condition such as rheumatoid arthritis, the compounds could be injected directly into the affected joint.

For the orally administered pharmaceutical compositions and methods of the present invention the foregoing active ingredients will typically be administered in admixture with suitable pharmaceutical diluents, excipients, or carriers (collectively referred to herein as "carrier" materials) suitably selected with respect to the intended form of administration, that is, oral tablets, capsules, softgels, elixirs, syrups, drops, and the like, and consistent with conventional pharmaceutical practices.

For example, for oral administration in the form of tablets or capsules, the active drug components may be combined with any oral non-toxic pharmaceutically acceptable inert carrier such as lactose, starch, sucrose, cellulose, magnesium stearate, dicalcium phosphate, calcium sulfate mannitol, and the like, or various combinations thereof. For oral administration in liquid forms, such as in softgels, elixirs, syrups, drops and the like, the active drug components may be combined with any oral non-toxic pharmaceutically acceptable inert carrier such as water, saline, ethanol, polyethylene glycol, propylene glycol, corn oil, cottonseed oil, peanut oil, sesame oil, benzyl alcohol, various buffers, and the like, or various combinations thereof. Moreover, when desired or necessary, suitable binders, lubricants, disintegrating agents, and coloring agents can also be incorporated in the mixture. Suitable binders include starch, gelatin, natural sugars, corn sweeteners, natural and synthetic gums such as acacia, sodium alginate, carboxymethylcellulose, polyethylene glycol, and waxes, or combinations thereof. Lubricants for use in these dosage forms include boric acid, sodium benzoate, sodium acetate, sodium chloride, and the like, or combinations thereof. Sweetening and flavoring agents and preservatives can also be included where appropriate.

For intravascular, intraperitoneal, subcutaneous, intramuscular or aerosol administration, active drug components may be combined with a suitable carrier such as water, saline, aqueous dextrose, and the like. Regardless of the route of administration selected, the compounds of the present invention are formulated

into pharmaceutically acceptable dosage forms by conventional methods known to those skilled in the art. The compounds may also be formulated using pharmacologically acceptable base addition salts. Moreover, the compounds or their salts may be used in a suitable hydrated form.

Regardless of the route of administration selected, a non-toxic but therapeutically effective quantity of one or more compounds of this invention is employed in any treatment. The dosage regimen for preventing or treating inflammatory conditions with the compounds of this invention is selected in accordance with a variety of factors, including the type, age, weight, sex, and medical condition of the patient, the severity of the inflammatory condition, the route of administration, and the particular compound employed in the treatment. A physician or veterinarian of ordinary skill can readily determine and prescribe the effective amount of the drug required to prevent or arrest the progress of the condition. In so proceeding, the physician or veterinarian could employ relatively low doses at first and subsequently increase the dose until a maximum response is obtained.

The compounds of this invention are prepared by the general methods illustrated in Schemes A - F. In the discussion of these schemes, the conventional numbering of the naphthelene ring is employed as illustrated below.

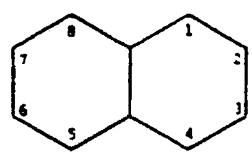

As illustrated in Scheme A, 7-methoxy-1-tetralone (X) is converted to the 2-enolate anion by a strong base, preferably NaH. Reaction of the 2-enolate anion with dimethyl carbonate produces carbomethylation of the 2-position (XI). Complete reduction of 1-oxo group of Compound XI is accomplished by hydrogenation over 5% Pd/C, resulting in a tetrahydronaphthalene compound (XII). Upon basic hydrolysis of the carbomethoxy ester moiety of XII, the 7-methoxyl group is converted to a 7-hydroxyl group by heating in pyridine HCl. Subsequent re-esterification results in 7-hydroxy-2-carbomethoxy-tetrahydro-naphthalene, represented by XIII. Conversion of the 7-hydroxyl group to its anion by reaction with a base, preferably $K_2CO_3$, and reaction of the anion with 2-propenyl bromide (allyl bromide) produces O-alkenylation (XIV) via nucleophilic substitution. Heating the ether (XIV) in diethyl aniline produces dealkenylation of the ether and selective alkenylation of the naphthyl ring at the 6 and 8 positions - ortho to the ether - producing the isomers XV and XVI. The 2-propenyl group at the 6 or 8 position is catalytically hydrogenated to an n-propyl group at the 6 position (XVIII) or 8 position (XVII) respectively, preferably using 5% Pd/C as catalyst.

In Scheme B, the 6-propyl (XVIII) and 8-propyl (XVII) isomers are converted into compounds claimed by the present invention. Specifically, the 7-hydroxyl group of XVIII and XVII are converted to respective anions by a base, preferably $K_2CO_3$, the anions of XVII and XVIII are reacted with a compound of the formula:

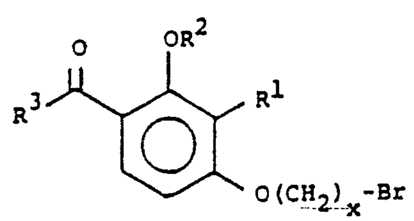

L

which is prepared according to U.S. Pat. 4,565,882, to produce compounds of the general formulas XIX and XX respectively. By varying $R^1$, $R^2$, $R^3$, and X, according to methods taught in the '882 patent and by methods known in the art, one can obtain all the variations necessary to produce the compounds of the present invention.

For example, the '882 patent teaches the method for producing a compound according to formula L, 3-(2-n-propyl-3-hydroxy-4-acetylphenoxy)-1-bromopropane, wherein $R^1$ is n-propyl, $R^2$ is -H, $R^3$ is methyl and X is 3. Reaction of the above compound with the anions of XVII and XVIII produces the compounds of Examples 9 and 14 of the present invention respectively.

Similarly, the '882 patent teaches the method for producing 5-(2-n-propyl-3-hydroxy-4-acetylphenoxy)-1-bromo-pentane, wherein $R^1$, $R^2$ and $R^3$ are as above but where X is 5. Reaction of this latter compound

with the anion of XVII produces the product of Example 10 of the present invention.

Basic hydrolysis of the methyl esters of XIX and XX is preferably accomplished by LiOH in aqueous/alcohol and produces the corresponding carboxylic acids XXI and XXII. Moreover either the carboxylic acids or the esters just described (XIX and XX) can be further reacted with an alcohol, $R^9$-OH, by methods known in the art to produce the corresponding esters (-$COOR^9$) of the present claimed invention.

In Scheme C, the same starting material as in Scheme A, 7-methoxy-1-tetralone (X), is demethylated by heating in pyridine HCl to produce the 7-hydroxy derivative (XXX). Conversion of the 7-hydroxyl group of compound (XXX) to its anion by a base, preferably $K_2CO_3$, and subsequent reaction with 2-propenyl bromide (allyl bromide) produces an O-alkenylation product (XXXI) similar to formulas XV and XVI in Scheme A.

Upon heating in an inert atmosphere, the previously alkenylated ether (XXXI) dealkenylates at the ether moiety while selectively alkenylating the naphthyl ring predominantly ortho to the C-7 ether, at the C-8 position, producing XXXII. Compound XXXII is then hydrogenated to produce the corresponding 8-propyl-7-hydroxy-1-tetralone (XXXIII).

The 7-hydroxyl group of XXXIII is converted (protected) to the benzyl ether (XXXIV) by reaction with benzyl bromide in the presence of a suitable base, preferably $K_2CO_3$. In the presence of a strong base, preferably NaH, XXXIV forms an enolate anion at C-2 of the tetralone ring. Reaction of the enolate anion with diethyl carbonate produces carboethoxylation at the C-2 position of the 1-tetralone ring structure (XXXV).

Upon reaction of Compound XXXV with a strong base, preferably NaH, a new enolate anion is formed at C-2. This latter enolate anion then adds to the double bond of an unsaturated carboxylic ester, such as ethyl 2-propenoate, to produce the corresponding diester (XXXVI).

Reaction of the diester (XXXVI) with acetic acid/HCl and methylorthoformate produces both cleavage of the benzyl protecting group and decarboxymethylation of the $\beta$-keto acid resulting in two products: the mono carboxylic ester (XXXVII) and the trapped enol ether (XXXVIII).

In Scheme D, the 7-hydroxyl groups of XXXVII and XXXVIII are converted to their respective anions by a base, preferably $K_2CO_3$. The respective anions can then be reacted with either an excess of 1,3-dibromopropane, 1,4-dibromobutane, or 1,5-dibromo pentane to produce the corresponding bromo-ethers of formulas XXXIX and XL. Subsequent reaction of XXIX and XL individually with 2,4-dihydroxy-3-n-propylacetophenone prepared according to U.S. Pat. 4,565,882, and excess $K_2CO_3$ produces claimed compounds of formulas XLI and XLII respectively.

Hydrolysis of the ester moieties on XLI and XLII by LiOH in aqueous methanol produces the corresponding carboxylic acids XLIII and XLIV respectively.

In Scheme D, when the dibromo compound is 1,3-dibromopropane, (X = 3), then Compounds XLI and XLII correspond to Examples 36 and 37 and Compounds XLIII and XLIV correspond to Examples 38 and 39 respectively of the present invention.

In Scheme E, the carbomethoxy moiety at C-2 of compound XVII is reduced to the corresponding 2-hydroxymethyl (XLV) by $LiBH_4$ in an aprotic solvent, preferably tetrahydrofuran (THF). Oxalyl chloride/dimethyl sulfoxide/triethylamine selectively oxidizes the C-2 hydroxymethyl group of XLV to the corresponding 2-carboxaldehyde (XLVI). Compound XLVI then reacts with the enolate anion of trimethyl phosphoacetate, which is prepared in situ in the presence of the strong base, preferably NaH, in an aprotic solvent, to produce a mixture of the trans and cis aldehyde addition products -Compounds XLVII and XLVIII respectively.

The 7-hydroxyl groups of XLVII and XLVIII are converted to their respective anions by a base, preferably an alkali metal carbonate, more preferably $K_2CO_3$. The respective anions are then reacted with either an excess of 1,3-dibromopropane, 1,4-dibromobutane or 1,5-dibromopentane to produce the corresponding bromoethers of general formulas XLIX and LI. Subsequent reaction of XLIX and LI with the phenoxide anion of L (U.S. Pat. 4,565,882) produces the diethers LII and LIII respectively.

In Compounds LII and LIII above, when $R^1$ is n-propyl, $R^2$ is hydrogen, $R^3$ is methyl, and X is 3, Compound LII corresponds to the product of Example 20 of the present invention and Compound LIII corresponds to the product of Example 21.

Scheme F is a continuation of Scheme E showing the further conversion of LII and LIII into compounds of this invention. More specifically, the ester moieties of LII and LIII are hydrolyzed to the corresponding carboxylic acids, LIV and LV, by LiOH in an aqueous/alcohol solvent. In another reaction, the alpha-beta unsaturation in the alkenyl ester (LII) is hydrogenated over 5% Pd/C to produce (LVI) bearing the corresponding alkylester side chain.

SCHEME A

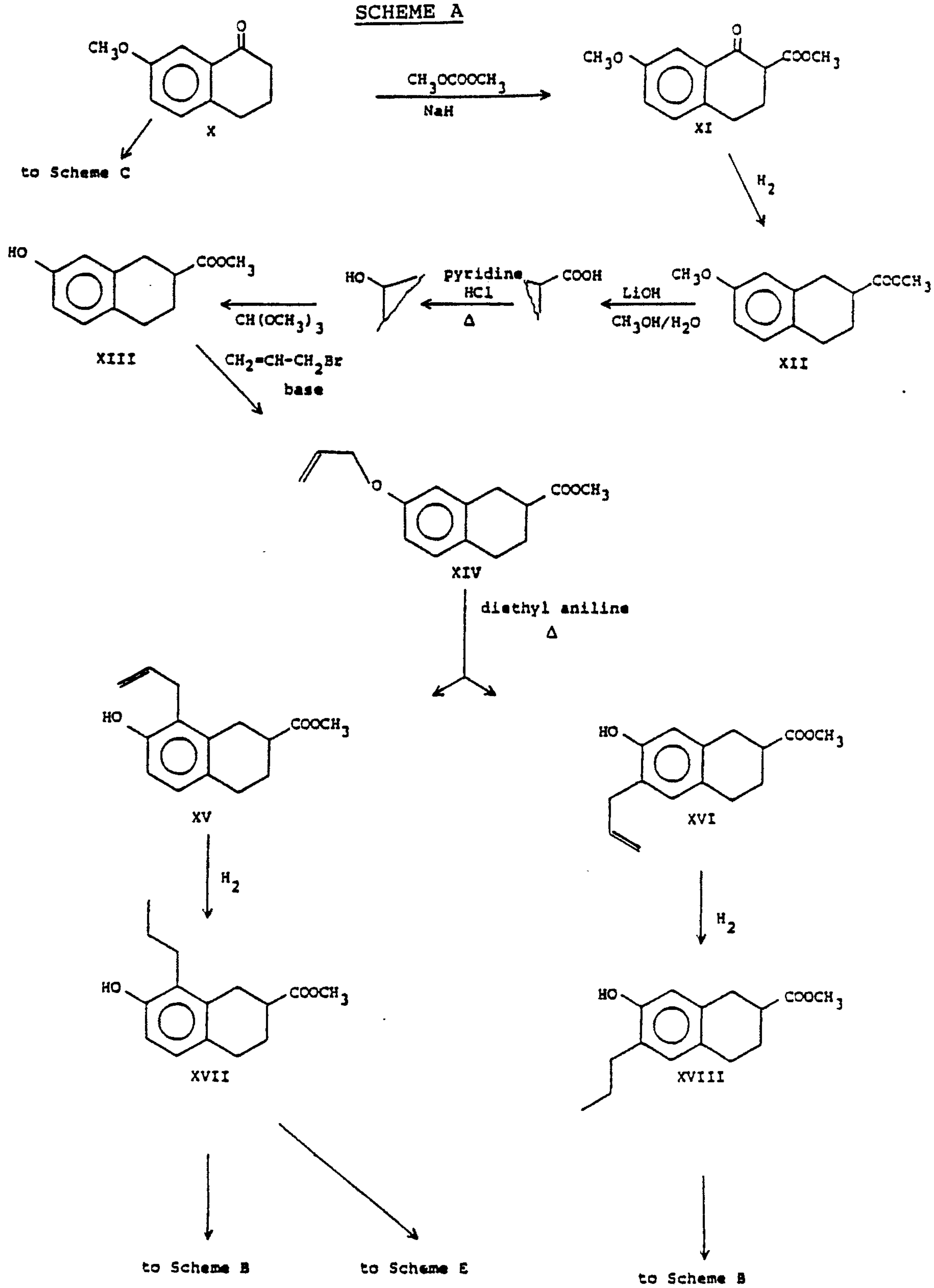

$CH_3OCOOCH_3$
NaH
X
XI
to Scheme C
$H_2$
pyridine
HCl
Δ
LiOH
$CH_3OH/H_2O$
$CH(OCH_3)_3$
XIII
XII
$CH_2=CH-CH_2Br$
base
XIV
diethyl aniline
Δ
XV
XVI
$H_2$
$H_2$
XVII
XVIII
to Scheme B
to Scheme E
to Scheme B

SCHEME B

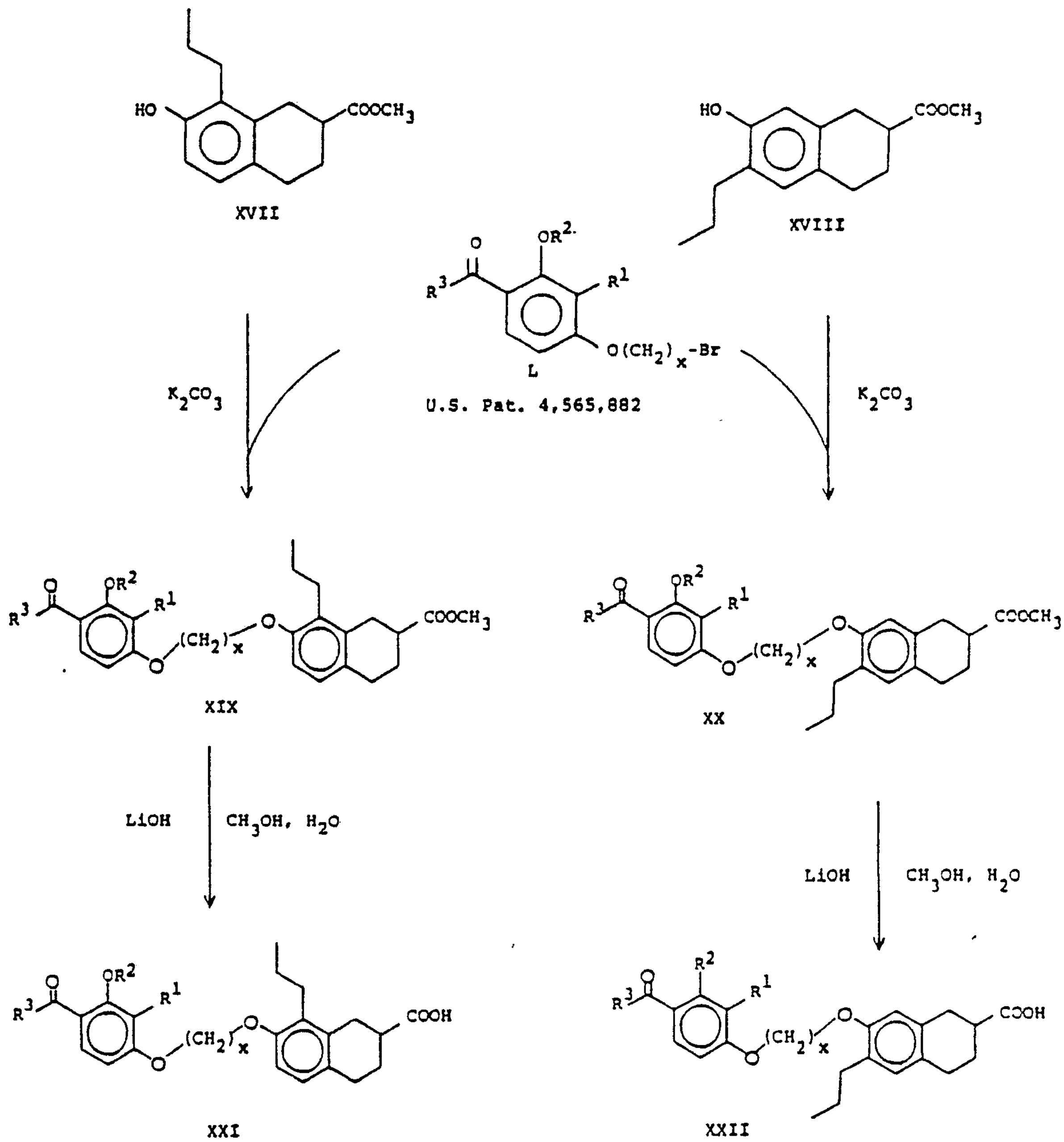
XVII
XVIII
L
U.S. Pat. 4,565,882
$K_2CO_3$
$K_2CO_3$
XIX
XX
LiOH
$CH_3OH$, $H_2O$
LiOH
$CH_3OH$, $H_2O$
XXI
XXII

SCHEME C

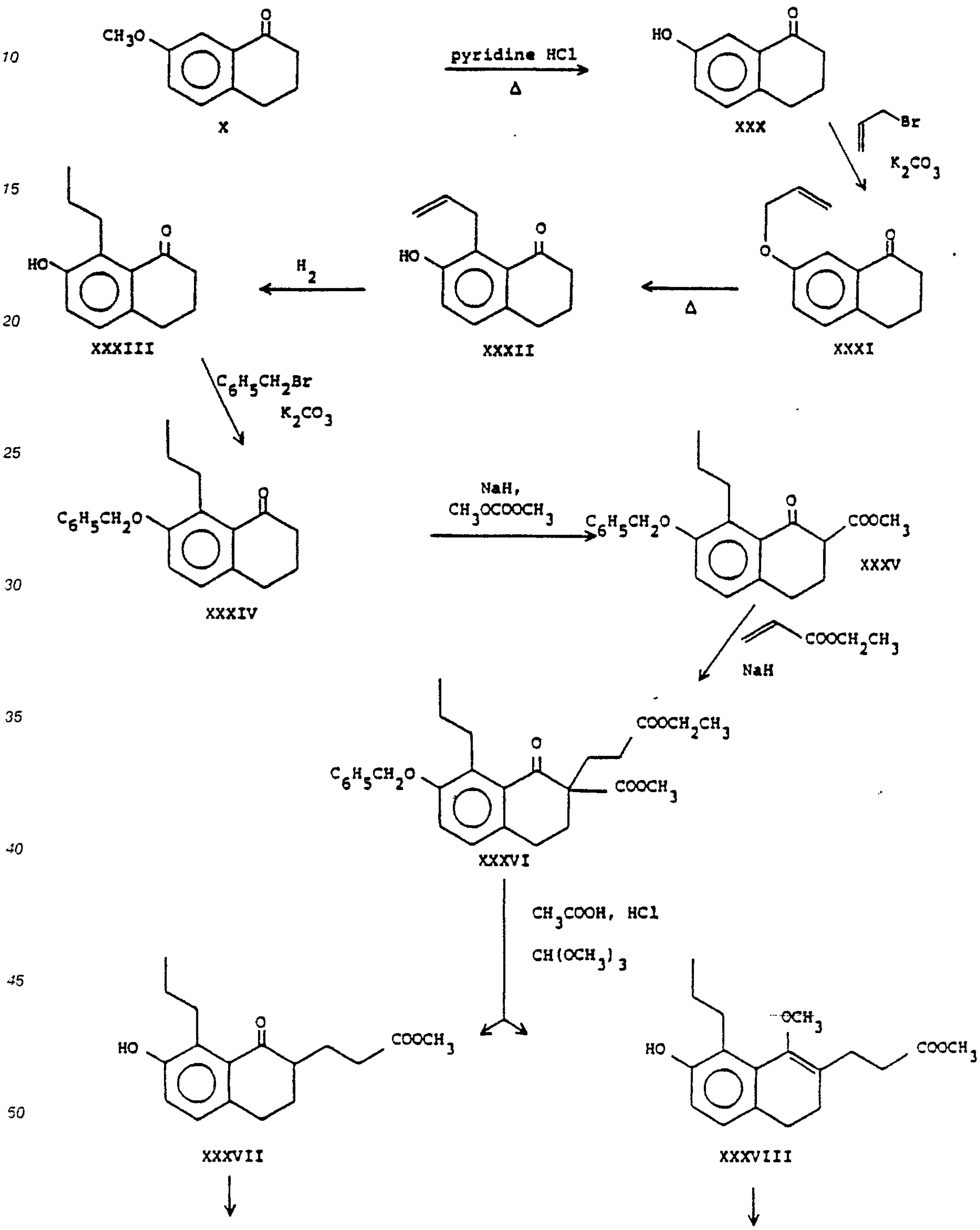


X

XXX

XXXI

XXXII

XXXIII

XXXIV

XXXV

XXXVI

XXXVII

XXXVIII

to Scheme D

to Scheme D

SCHEME D

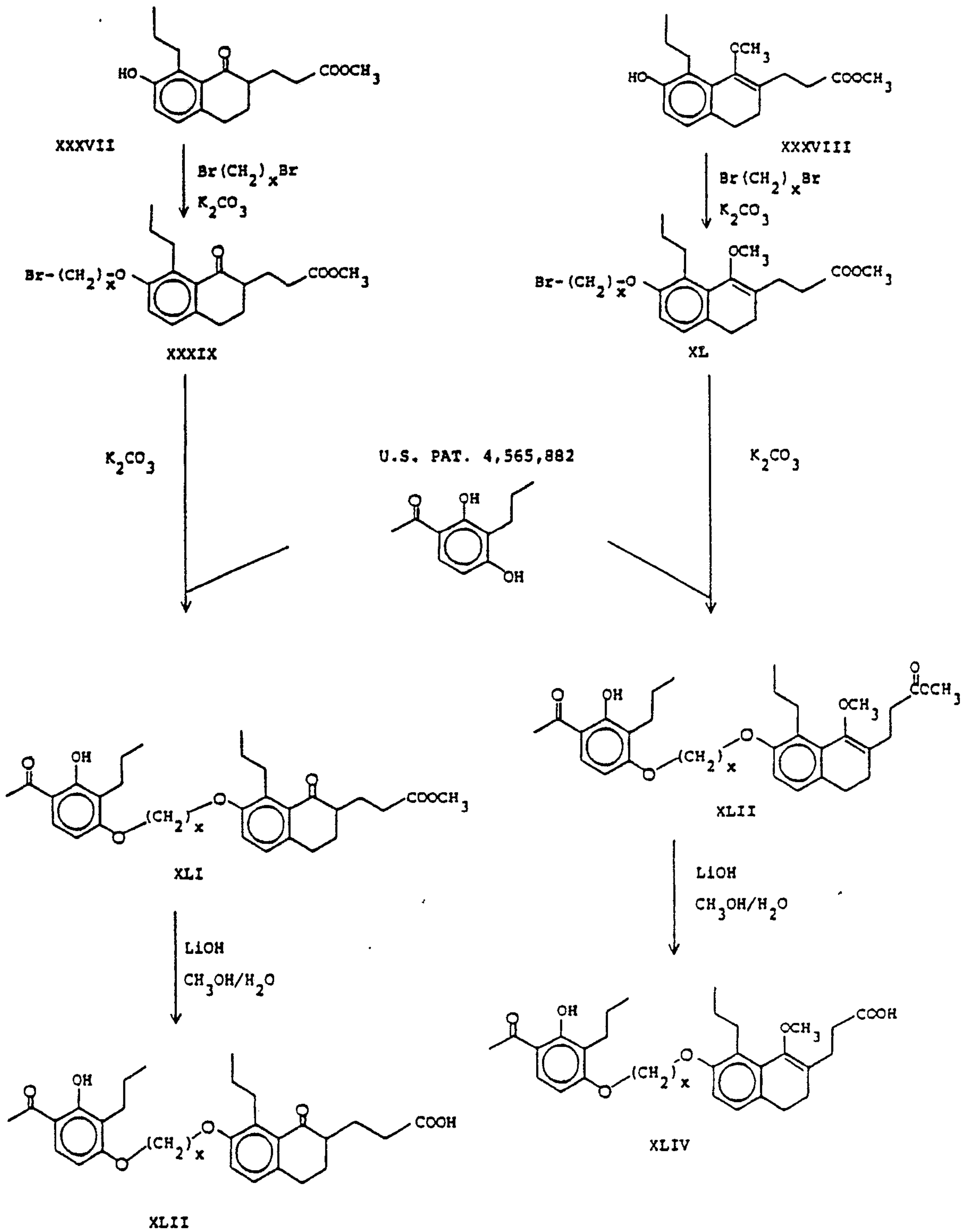
XXXVII
XXXVIII
Br(CH2)xBr
K2CO3
Br(CH2)xBr
K2CO3
XXXIX
XL
K2CO3
U.S. PAT. 4,565,882
K2CO3
XLI
XLII
LiOH
CH3OH/H2O
LiOH
CH3OH/H2O
XLIV
XLII

SCHEME E

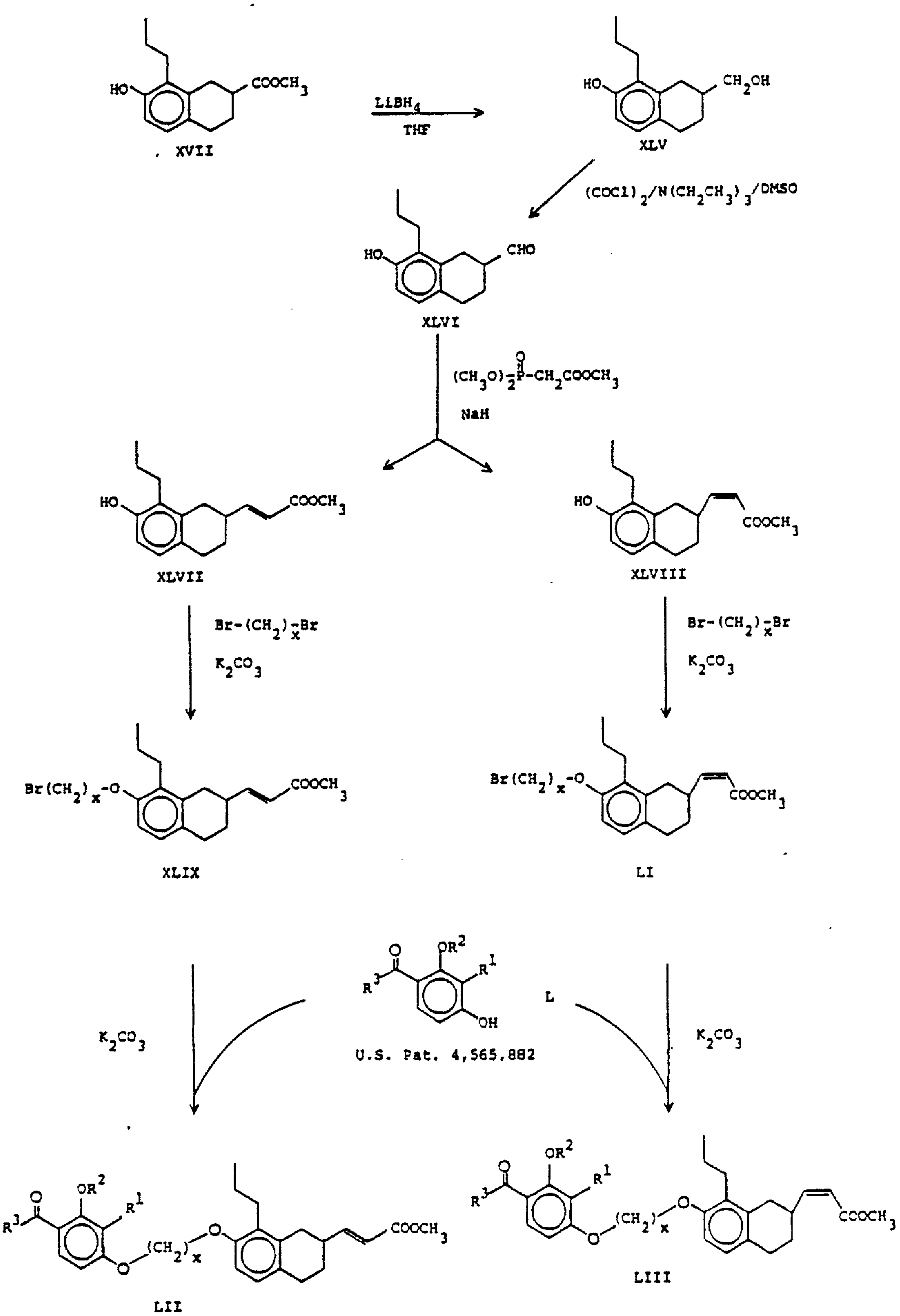


XVII, XLV, XLVI, XLVII, XLVIII, XLIX, LI, L, U.S. Pat. 4,565,882, LII, LIII

SCHEME F

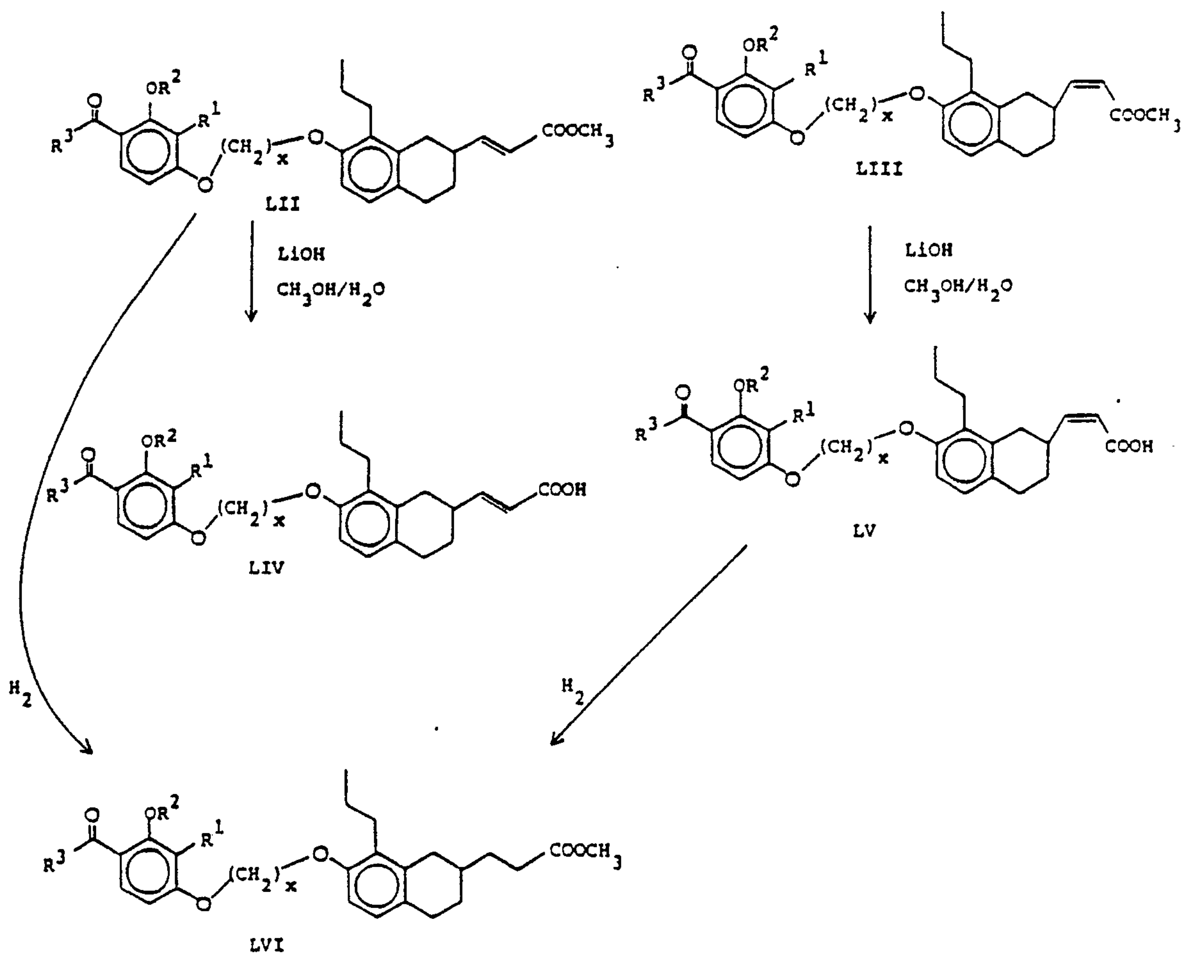


## DESCRIPTION OF THE PREFERRED EMBODIMENT

### Example 1

methyl 1,2,3,4-tetrahydro-7-methoxy-1-oxo-2-naphthalene carboxylate

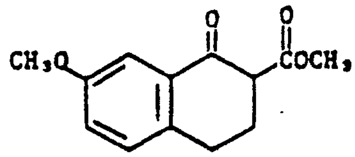

A 50% slurry of NaH (30g, 0.62 moles) in hexane was filtered through a fritted glass funnel to remove the mineral. The NaH was then added to a 2L flask, covered with 300ml of tetrahydrofuran (THF), and placed under a $N_2$ atmosphere. Upon the addition of 0.62 moles of dimethyl carbonate at one time with stirring, the reaction mixture was heated to 40°-50° C, whereupon 50g (0.28 moles) of commercially available 7-methoxy-1-tetralone in 150ml of THF was added at a rate to minimize foaming (1 hr.). After refluxing the reaction mixture for 2 hrs., the solution (red) was cooled to room temperature (R.T.) and slowly acidified by the addition of 45ml of acetic acid. The resulting paste was dissolved upon addition of 50ml of water. Ether was added and the layers were separated. The organic phase was washed with $H_2O$, 3% $NaHCO_3$ solution, and dried ($Na_2SO_4$). After filtration, the solvent was evaporated on a rotary evaporator. The residue was distilled at 168°-170° C at .2mm Hg.

Analysis for $C_{13}H_{16}O_4$ (MW = 236.26):
Calcd: C, 66.66; H, 6.02.
Found: C, 66,87; H, 6.07.

Example 2

methyl 1,2,3,4-tetrahydro-7-methoxy-2-naphthalenecarboxylate

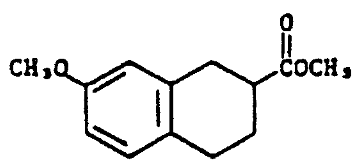

To 58g (.248 mole) of the titled product of Example 1 dissolved in a mixture of 387ml of acetic acid and 16.6ml of perchloric acid and placed in a 1L pressure bottle was added 5.8g of 5% Pd/C. The bottle was placed in a Parr shaker and hydrogenated at R.T. at 30 p.s.i. for 2 hr. After filtration, the filtrate was diluted with 1.1 liter of $CHCl_3$, washed with $H_2O$ until the pH was neutral (5x). The organic phase was dried ($Na_2SO_4$) and the solvent evaporated to produce 49g of a crude yellow oil (the titled product).

Analysis fo $C_{13}H_{16}O_3$ (MW = 220.26):
Calcd: C, 70.88; H, 7.32.
Found: C, 70.62; H, 7.09.

Example 3

1,2,3,4-tetrahydro-7-methoxy-2-naphthalenecarboxylic acid

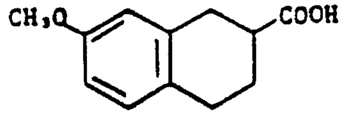

To 49g (0.22 mole) of the product of Example 2 dissolved in 600ml of methanol at R.T. was added 150ml of 2M LiOH with stirring. After heating the stirred solution at 50°C for 2 hr, the methanol was removed by rotary evaporation, the aqueous residue was washed 1x with ether, and acidified to pH 2 with HCl. Upon cooling and stirring, the crude acid precipitated and was separated by filtration. The crude acid was recrystallized from ether-hexane to give 30g (67%) of the titled product, m.p. 119°-122°C.

Analysis for $C_{12}H_{14}O_3$ (MW = 206.23):
Calcd: C,69.89; H, 6.84.
Found: C, 69.70; H, 6.92.

Example 4

1,2,3,4-tetrahydro-7-hydroxy-2-naphthalenecarboxylic acid

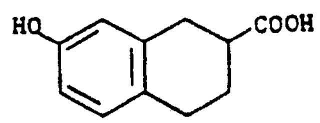

To a 500ml flask containing 100g of pyridine hydrochloride was added 30g of the product of Example 3 and the reaction mixture was blanketed with $N_2$. The reaction mixture was placed on an oil bath and heated to 215°C for 2 hr. Upon cooling to R.T., 200ml of 1N HCl was added with stirring followed by 300ml of a 1:1 mixture of ether/ethyl acetate. The organic phase was separated, washed 3x with $H_2O$, and dried ($MgSO_4$), and filtered. Upon evaporation of the solvent, the residue was recrystallized from ether/hexane to produce 21.4g of the titled product, m.p. 170°-171°C.

Analysis for $C_{11}H_{12}O_3$ (MW = 192.21):
Calcd: C, 68.73; H, 6.29.
Found: C, 68.58; H, 6.35.

Example 5

methyl 1,2,3,4-tetrahydro-7-hydroxy-2-naphthalenearboxylate

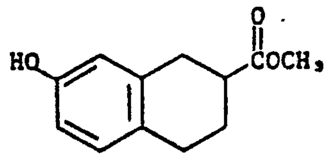

To 21g of the product of Example 4 dissolved in 250ml of methanol and placed in a 1L flask was added 30ml of trimethylorthoformate and 8ml of $H_2SO_4$. The flask was covered and allowed to stand at room temperature for 3 days. Upon rotary evaporation of the methanol, 500ml of ether was added and the organic phase was washed 2x with 3% $NaHCO_3$ solution, and 2x with water. The organic phase was dried ($Na_2SO_4$), filtered, and the solvent removed by rotary evaporation. The residue was recrystallized from ethyl acetate-hexane to give 22g of the titled product, m.p. 83-84° C.

Analysis for $C_{12}H_{14}O_3$ (MW = 206.24):
Calcd: C, 69.89; H, 6.84.
Found: C, 69.86; H, 6.82.

Example 6

methyl 1,2,3,4-tetrahydro-7-(2-propenyloxy)-2-naphthalenecarboxylate

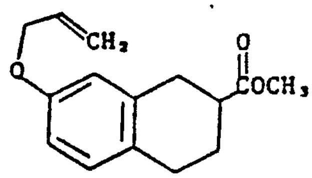

To 20g (0.097 mole) of the product of Example 5 dissolved in 300ml of dry acetone was added 35g (2.5 eq.) of $K_2CO_3$ with vigorous stirring. An excess (20g) of allyl bromide was then added to the reaction vessel and the mixture was refluxed with a drying tube attached for 1 day. The reaction mixture was filtered and the solvent in the filtrate evaporated by rotary evaporation. The residue was taken up in 50ml of toluene and chromatographed on a 500g silica gel flash column, eluting with toluene. The eluent was evaporated and 17.5g (78%) of the titled product was isolated as a clear oil.

Analysis for $C_{15}H_{18}O_3$ (MW = 246.31):
Calcd: C, 73.15; H, 7.37.
Found: C, 72.84; H, 7.44.

Example 7

methyl 1,2,3,4-tetrahydro-7-hydroxy-8-(2-propenyl)-2-naphthalenecarboxylate

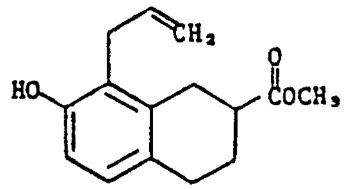

To 20g of diethylaniline in a reaction vessel was added under argon 17g of the product of Example 6. After the reaction mixture was heated at 215° C for 6 hr, thin-layer chromatography (TLC) showed that two products had formed. Upon cooling, the reaction contents were poured into a 3:1 mixture of ethyl acetate/ether and the resulting solution washed with 200ml of 2N HCl and 100ml of water. The organic layer was separated, dried ($Na_2SO_4$), and filtered. Upon removal of the solvent by rotary evaporation, the crude residue (17g) was chromatographed on silica gel, eluting with 3% acetone/toluene. The most non-polar material (12.5g) was the titled product, m.p. 90-91° C. The most polar material was methyl 1,2,3,4-tetrahydro-7-hydroxy-6-(2-propenyl)-2-naphthalenecarboxylate.

Analysis for $C_{15}H_{18}O_3$ (MW = 246.31):
Calcd: C, 73.15; H, 7.37.
Found: C, 73.01; H, 7.34.

Example 8

methyl 1,2,3,4-tetrahydro-7-hydroxy-8-propyl-2-naphthalenecarboxylate

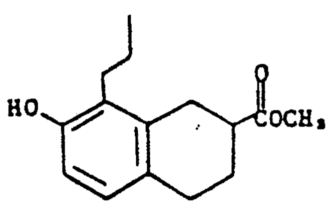

To 12g of the product of Example 7 dissolved in 125ml of methanol and placed in a pressure bottle was added 1.25g of 5% Pd/C. The bottle was placed in a Parr shaker and the mixture hydrogenated at room temperature and 2 p.s.i. for 1 hr. Upon filtration of the reaction mixture and evaporation of the solvent, 12g of the titled product was produced as an oil.

Analysis for $C_{15}H_{20}O_3$ (MW = 248.32):
Calcd: C, 72.55; H, 8.12.
Found: C, 71.83; H, 8.20.

Example 9

methyl 7-[3-(4-acetyl-3-hydroxy-2-propylphenoxy)propoxy]-1,2,3,4-tetrahydro-8-propyl-2-naphthalenecarboxylate

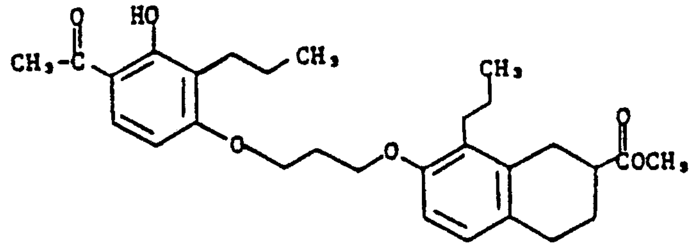

To 1.6g (.005 moles) of 3-(2-n-propyl-3-hydroxy-4-acetylphenoxy)-1-bromopropane prepared as described in Miyano et. al. (U.S. Pat. 4,565,882) and dissolved in 30ml of methyl ethyl ketone was added 2g (2.5 eg.) of $K_2CO_3$, 1.25g (.005 mole) of the product of Example 8, and 100mg of NaI. The reaction mixture was placed under a $N_2$ atmosphere and the contents refluxed for 48 hr. Upon cooling, the reaction mixture was filtered and the filtrate evaporated to produce a residue that was chromatographed on a silica gel column and eluted with 3% acetone/toluene. Evaporation of the eluent produced 1.45g of the titled compound, m.p. 84°-85° C.

Analysis for $C_{29}H_{38}O_6$ (MW = 482.62):
Calcd: C, 72.17; H, 7.94.
Found: C, 71.87; H, 7.90.

## Example 10

methyl 7-[5-(4-acetyl-3-hydroxy-2-propylphenoxy)pentyloxy]-1,2,3,4-tetrahydro-8-propyl-2-naphthalenecarboxylate

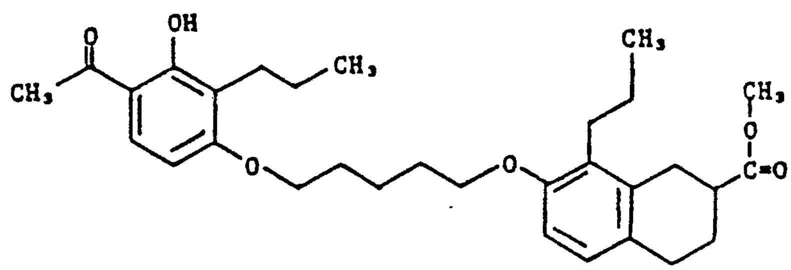

To 3.4g (.001 mole) of 5-(2-n-propyl-3-hydroxy-4-acetylphenoxy)-1-bromopentane prepared as described in U.S. Pat. 4,565,882 and dissolved in 60ml of methyl ethyl ketone containing 4g (2.5 e.q.) of $K_2CO_3$ and 2.5g (.001 mole) of the product of Example 8 was added 100mg of NaI. The reaction mixture was then refluxed under Ar for 3 days. Upon cooling, the reaction mixture was filtered and the filtrate evaporated down to a residue. After chromatography of the residue on silica gel, eluting with 3% acetone-toluene, the eluent yielded 3g of the titled product as a clear oil upon evaporation of the solvent.

Analysis for $C_{31}H_{42}O_6$ (MW = 510.68):
Calcd: C, 72.91; H, 8.29.
Found: C, 72.42; H, 8.24.

## Example 11

7-[3-(4-acetyl-3-hydroxy-2-propylphenoxy)propoxy]-1,2,3,4-tetrahydro-8-propyl-2-naphthalenecarboxylic acid

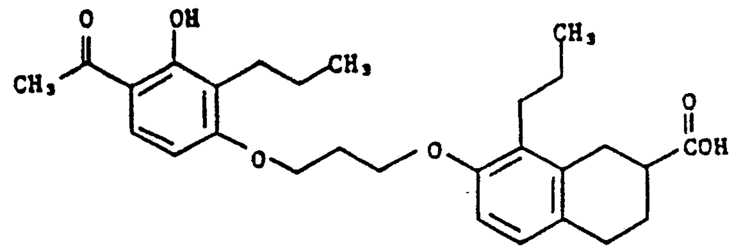

To 1g of the product of Example 9 dissolved in 20ml of methanol was added 3ml of 2M lithium hydroxide (2.5 eq.) and the reaction mixture was stirred at room temperature under $N_2$ for 5 hr. Upon removal of the methanol, the aqueous residue was acidified by 0.5N $KHSO_4$ to pH 2 and a precipitate formed. The precipitate was filtered, air dried, and recrystallized from 2B ethanol to produce 602mg of the titled material, m.p. 135°-137° C.

Analysis for $C_{28}H_{36}O_6$ (MW = 468.57):
Calcd: C, 71.77; H, 7.74.
Found: C, 71.74; H, 7.71.

## Example 12

7-[5-(4-acetyl-3-hydroxy-2-propylphenoxy)pentyloxy]-1,2,3,4-tetrahydro-8-propyl-2-naphthalenecarboxylic acid

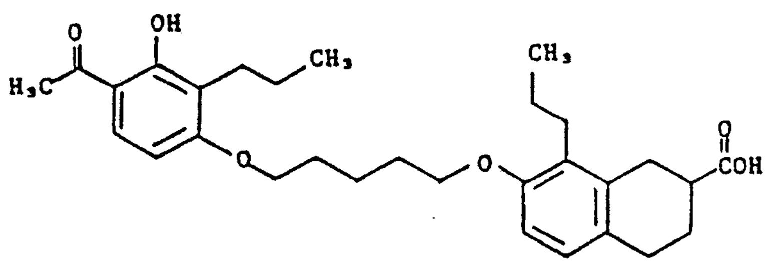

To 3g of the product of Example 10 dissolved in 60ml of methanol was added 10ml of 2M LiOH (2.5 eq.) and the reaction mixture was allowed to stand at room temperature under $N_2$ for 6 hr. Upon removal of the methanol by rotary evaporation, the aqueous residue was acidified to pH 2 with 0.5N $KHSO_4$. The semi-solid that separated was extracted with ether, washed with water, dried ($Na_2SO_4$) and filtered. The ether was removed by rotary evaporation and the residue was recrystallized from ethyl acetate-hexane to yield 2.8g of the titled material, m.p. = 106°-108° C.

Analysis for $C_{30}H_{40}O_6$ (MW = 496.62):
Calcd: C, 72.55; H, 8.12.
Found: C, 72.53; H, 8.08.

Example 13

methyl 1,2,3,4-tetrahydro-7-hydroxy-6-propyl-2-naphthalenecarboxylate

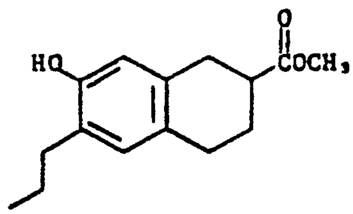

To 3g of the most polar material from Example 7, methyl 1,2,3,4-tetrahydro-7-hydroxy-6-(2-propenyl)-2-naphthalenecarboxylate, dissolved in 100ml of methanol in a pressure bottle was added 300mg of 5% Pd/C. The bottle was placed on a Parr shaker and hydrogenated at room temperature at 2 p.s.i. for 3 hr. The reaction mixture was filtered and the solvent removed by rotary evaporation. The residue was recrystallized from ethyl acetate-hexane to give 3g of the titled material, m.p. 114°-115°C.

Analysis for $C_{15}H_{20}O_3$ (MW = 248.3):
Calcd: C, 72.55; H, 8.12.
Found: C, 72.52; H, 8.04.

Example 14

methyl 7-[3-(4-acetyl-3-hydroxy-2-propylphenoxy)propoxy]-1,2,3,4-tetrahydro-6-propyl-2-naphthalenecarboxylate

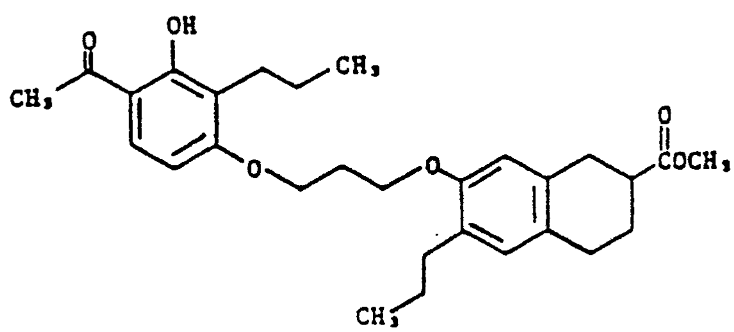

To 1.25g (.005 mole) of the product of Example 13 dissolved in 25ml of methyl ethyl ketone was added 1.6g (.005 mole) of 3-(2-n-propyl-3-hydroxy-4-acetylphenoxy)-1-bromopropane which was prepared according to U.S. Pat. 4,565,882. Upon the addition of 100mg of NaI, the reaction mixture was refluxed for 3 days under an Ar blanket. The reaction mixture was cooled to room temperature, filtered, and the solvent removed by rotary evaporation to give a crude residue. The residue was chromatographed on silica gel eluting with 1% acetone/toluene. Evaporation of the solvent and recrystallization from cyclohexane yielded 1.5g of the titled material, m.p. 60°-62°C.

Analysis for $C_{29}H_{38}O_6$ (MW = 482.62):

Calcd: C, 72.17; H, 7.94.
Found: C, 72.17; H, 8.00.

Example 15

7-[3-(2-propyl-3-hydroxy-4-acetylphenoxy)propoxy]-1,2,3,4-tetrahydro-6-propyl-2-naphthalenecarboxylic acid

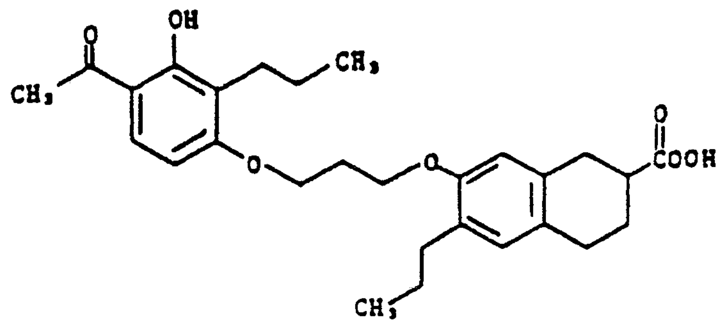

To 1.25g of the product of Example 14 dissolved in 60ml of methanol was added 5ml of 2M LiOH and the reaction mixture was stirred at 45° C for 2 hr. Afterwards, the solvent was removed by rotary evaporation and the aqueous residue was acidified to pH 2 by addition of 0.5N $KHSO_4$. The product, which precipitated out of solution, was filtered and air dried. Recrystallization from cyclohexane produced 1.1g of the titled material, m.p. 86°-90° C.

Analysis for $C_{28}H_{36}O_6$ (MW = 468.57):
Calcd: C, 71.77; H, 7.74.
Found: C, 72.28; H, 7.94.

Example 16

1,2,3,4-tetrahydro-7-hydroxy-8-propyl-2-naphthalenemethanol

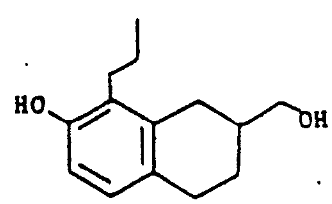

To 1.4g (0.064 mole) of $LiBH_4$ in 200ml of dry THF under a blanket of Ar in a 1L 3-necked flask was added slowly over a 30 min. period 8g (0.32 mole) of the product of Example 8. The reaction mixture was stirred for 2 hr. at room temperature and then refluxed for 2 days. After cooling to room temperature, 25 ml of water was slowly added followed by 15ml of IN HCl. Ether was then added to the reaction mixture and the layers separated. The organic phase was dried ($MgSO_4$), filtered, and the solvent evaporated. The residue was chromatographed on silica gel, eluting with 10% acetone-toluene. Evaporation of the solvent produced 5g of the titled material as an oil.

Analysis for $C_{14}H_{20}O_2$ (MW = 220.31):
Calcd: C, 76.33; H, 9.15.
Found: C, 76.07; H, 9.11.

Example 17

1,2,3,4-tetrahydro-7-hydroxy-8-propyl-2-naphthalenecarboxaldehyde

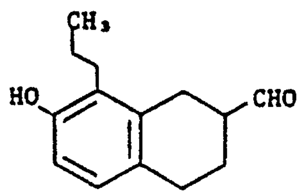

To 75ml of dry $CH_2Cl_2$ cooled under $N_2$ to -60°C was added 4ml (44 mmol) of oxalyl chloride. The temperature was allowed to climb to -50°C and 5.8ml (88 mmol) of dry DMSO was added over a 5 min. period. Then, 4.25g (19.3 mmol) of the product of Example 16 dissolved in 35ml of DMSO was added at -50°C over 10 min. To effect solution, the reaction mixture was allowed to warm to -30°C for 30 min. whereupon it was recooled to -50°C and 15ml (100 mmol) of freshly distilled triethyl amine was added over 5 min. After the mixture was stirred at -50°C for 15 min., it was allowed to warm up to room temperature over 1 hr. and then 50ml of water was added. The layers were separated and the organic phase was washed with 50ml of 0.25N $NaHSO_4$, and then water. The organic phase was dried ($MgSO_4$), filtered, and solvent evaporated to produce an oily residue. Chromatography on silica gel, eluting with 2% acetone/toluene produced 3.5g of the titled material in the major fraction. Rechromatography with 12% ethyl acetate/hexane produced a fraction yielding crystals, m.p. 93°-94°C.

Analysis for $C_{14}H_{18}O_2$ (MW = 218.28):
Calcd: C, 77.03; H, 8.31.
Found: C, 76.76; H, 8.30.

Example 18

methyl 3-[1,2,3,4-tetrahydro-7-hydroxy-8-propyl-2-naphthalenyl]-2E/Z-propenoate

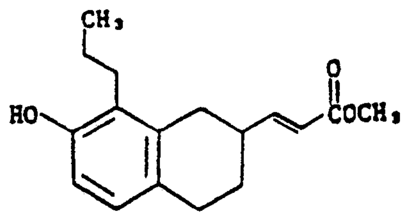

**only E (trans) isomer is shown**

To 2.67g (.0146 moles) of trimethylphosphoacetate slurried in 50ml of dimethoxyethane (DME) and cooled to 0° C was added portionwise and under a $N_2$ blanket 360mg (.0146 mole) of NaH which had been obtained by washing 720mg of a 50% oil dispersion with hexane. The reaction was allowed to warm to room temperature and 1.6g (.007 mole) of the product of Example 17 in 10ml of DME was added at once. The reaction mixture was stirred at room temperature for 3 hr. at which time 30ml of water was added followed by 70ml of ethyl acetate. The organic phase was washed with brine, dried ($MgSO_4$), and filtered. Upon evaporation of the solvent 0.76g of a crude oil was obtained.

Analysis for $C_{17}H_{22}O_3$ (MW = 274.35):
Calcd: C, 73.42; H, 8.08.
Found: C, 73.16; H, 8.51.

Example 19

methyl 3-[7-(3-bromopropoxy)-1,2,3,4-tetrahydro-9-propyl-2-naphthalenyl]-2E/Z-propenoate

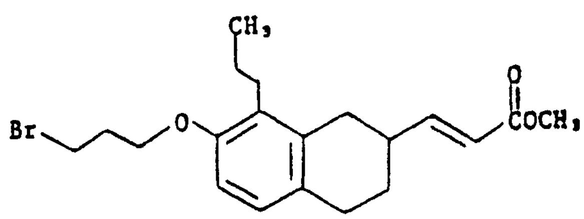

**only E (trans) isomer is shown**

To 1.76g of the crude material obtained in Example 18 and dissolved in 20ml of methyl ethyl ketone was added 1.76g (2 eq.) of $K_2CO_3$ and 20g (15 mole excess) of dibromopropane. After refluxing the reaction mixture under $N_2$ for 5 days, it was cooled to room temperature and filtered. The filtrate was stripped of its solvent, the residue was placed on a high vacuum pump (.1mm Hg), and the excess dibromopropane removed at 55° C. The residue was chromatographed on silica gel and eluted with 5% ethyl acetate-hexane producing two major fractions, the more non-polar material was identified as the Z (cis) isomer, 190mg as an oil. The more polar material was identified as the E (trans) isomer, 1.25g, m.p. 44°-45° C.

(trans isomer): E

Analysis for $C_{20}H_{27}O_3Br$ (MW = 395.34):
Calcd: C, 60.75; H, 6.88; Br, 20.21.
Found: C, 60.29; H, 6.99; Br, 20.57.

(cis isomer): Z

Analysis for $C_{20}H_{27}O_3Br$ (MW = 395.34)
Calcd: C, 60.75; H, 6.88; Br, 20.21.
Found: C, 60.58; H, 6.95; Br. 20.49.

## Example 20

methyl 3-[7-[3-(4-acetyl-3-hydroxy-2-propylphenoxy)propoxy]-1,2,3,4-tetrahydro-8-propyl-2-naphthalenyl]-2E-propenoate

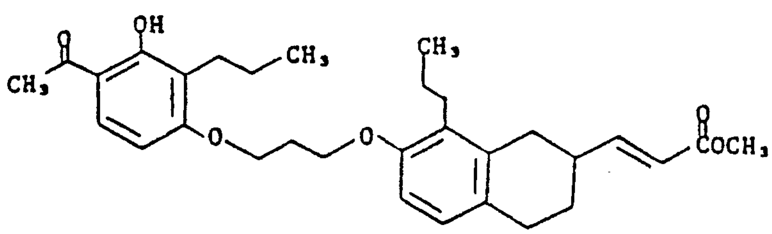

To 1g (.0025 mole) of the E (trans) isomer of Example 18 dissolved in 15ml of dimethylformamide was added 485mg (.0025 mole) of 2,4-dihydroxy-3-n-propylacetophenone and 700mg (2 eq.) of anhydrous $K_2CO_3$. After stirring for 24 hr. under a $N_2$ blanket, the reaction mixture was filtered and the filtrate placed under vacuum (0.1mm Hg). Chromatography of the residue on silica gel produced 1.6g of a crude solid when eluted with 15% ethyl acetate-hexane. Recrystallization from cyclohexane produced 1.1g of the titled product, m.p. 110°-111°C.

Analysis for $C_{31}H_{40}O_5$ (MW = 508.63):
Calcd: C, 73.20; H, 7.93.
Found: C, 73.29; H, 8.10.

## Example 21

methyl 3-[7-[3-(4-acetyl-3-hydroxy-2-propylphenoxy)propoxy]-1,2,3,4-tetrahydro-8-propyl-2-naphthalenyl]-2Z-propenoate

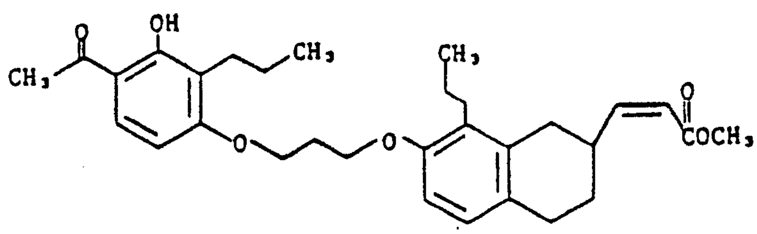

To 100mg (.0025 mole) of the Z(cis) isomer from Example 19 dissolved in 10ml of dimethylformamide (DMF) was added 48mg (.0025 mole) of 2,4-dihydroxy-3-n-propylacetophenone and 70mg (2 eq.) of anhydrous $K_2CO_3$. The reaction mixture was stirred at room temperature (R.T.) under a $N_2$ blanket for 24 hr. and then filtered and evaporation of the filtrate at 0.1mm Hg produced a residue which was chromatographed on silica gel. Elution with 1/2% acetone/toluene produced a fraction that yielded 75mg of a waxy solid, m.p. 25° C.

Analysis for $C_{31}H_{40}O_6$ (MW = 508.63):
Calcd: C, 73.20; H, 7.93.
Found: C, 73.03; H, 8.00.

Example 22

methyl 7-[3-(4-acetyl-3-hydroxy-2-propylphenoxy)propoxy]-1,2,3,4-tetrahydro-8-propyl-2-naphthalenepropanoate

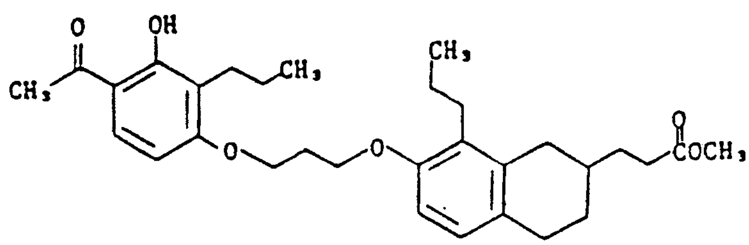

To 800mg of the E(trans) isomer from Example 20 dissolved in 25ml of THF was added 80mg of 5% Pd/C catalyst. Hydrogenation was conducted in a 100ml buret at room temperature and atmospheric pressure for 30 min. After filtration, the solvent was removed by rotary evaporation. The residue was recrystallized from cyclohexane to give 780mg of the titled compound, m.p. 69° -71° C.

Analysis for $C_{31}H_{42}O_6$(MW = 510.68)
Calcd: C, 72.26; H, 8.49.
Found: C, 72.34; H, 8.32.

Example 23

3-[7-[3-(4-acetyl-3-hydroxy-2-propylphenoxy)propoxy]-1,2,3,4-tetrahydro-8-propyl-2-naphthalenyl]-2E-propenoic acid

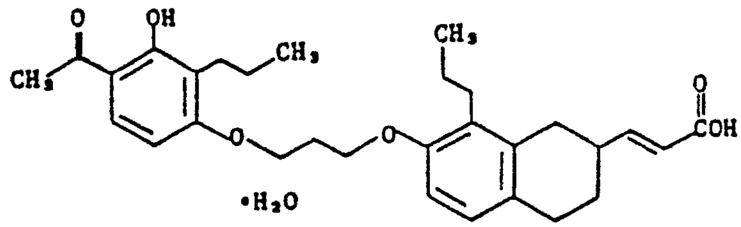

To 150mg of the E(trans) isomer from Example 20 dissolved in 15ml of methanol was added 2ml of 2M LiOH and the reaction mixture was allowed to stir at 50° C for 2 hr. Afterwards, the reaction was cooled to room temperature and the methanol removed via rotary evaporation. Upon acidification to pH 2 with 0.5N $NaHSO_4$ a precipitate formed, which was filtered and air dried. Recrystallization from ethyl acetate-hexane yielded 120mg of the titled material , m.p. 126°-129° C.

Analysis for $C_{30}H_{38}O_6 \bullet H_2O$ (MW = 512.62):
Calcd: C, 70.29; H, 7.87.
Found: C, 70.63; H, 7.83.

Example 24

7-[3-(4-acetyl-3-hydroxy-2-propylphenoxy)propoxy]-1,2,3,4-tetrahydro-8-propyl-2-naphthalenepropanoic acid

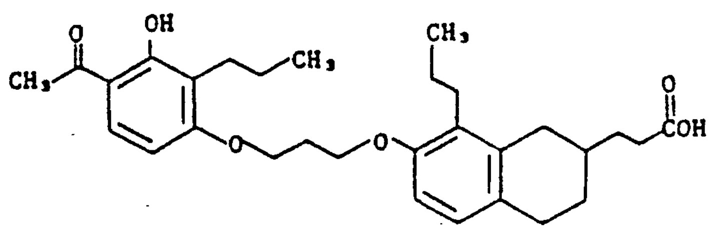

To 700mg of the titled material from Example 22 dissolved in 25ml of methanol was added two ml of 2M lithium hydroxide solution and the entire mixture was warmed to 45° C for 90 min. Upon cooling to room temperature, the methanol was removed from the reaction mixture by rotary evaporation. Upon acidification of the aqueous residue to pH 2 with 0.5N $NaHSO_4$, a precipitate formed, which was filtered and dried. Recrystallization from 2B ethanol yielded 500mg of the titled material, m.p. 111°-112° C.

Analysis for $C_{30}H_{40}O_6$ (MW = 496.62):
Calcd: C, 72.55; H, 8.12.
Found: C, 72.58; H, 8.37.

Example 25

3-[7-[3-(4-acetyl-3-hydroxy-2-propylphenoxy)propoxy]-1,2,3,4-tetrahydro-8-propyl-2-naphthalenyl]-2E-propenoic acid

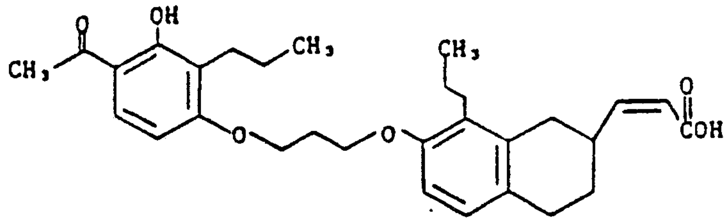

To 53mg (.001 mole) of the titled material from Example 21 dissolved in 15ml of methanol was added 2ml of 2M LiOH and the reaction mixture was warmed to 40° C for 3 hr. Upon cooling to room temperature, the methanol was removed by rotary evaporation. Upon acidification of the aqueous residue to pH 2 by 0.5N $NaHSO_4$, the oil which separated out, was extracted with ethyl acetate and diethyl ether. The organic layer was separated, dried ($MgSO_4$), and filtered. The solvent was blown off under a $N_2$ stream and the oil was diluted with hexane and placed in the refrigerator to yield 35mg of the titled maerial as crystals, m.p. 74°-77° C.

Analysis for $C_{30}H_{38}O_6$ $H_2O$ (MW = 512.62):
Calcd: C, 70.29; H, 7.87.
Found: C, 70.88; H, 7.94.

Example 26

3,4-dihydro-7-hydroxy-1[2H]-naphthalenone

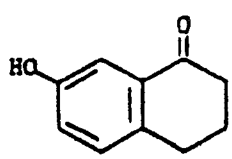

To 35g (.196 mole) of 3,4-dihydro-7-methoxy-1[2H]-naphthalenone in a round bottom flask was added 75g (0.6 mole) of pyridine hydrochloride. The reaction mixture was placed under a blanket of $N_2$ and heated to 220° C in an oil bath for 4 hr. Upon cooling to room temperature, the contents were diluted with water and extracted with ethyl acetate. The organic layer was washed with 2N HCl and the organic phase dried ($Na_2SO_4$) and filtered. The solvent was evaporated to produce a residue which was recrystallized from ethyl acetate/hexane to yield 21g of the titled material, m.p. 159°-162° C.

Analysis for $C_{10}H_{10}O_2$ (MW = 162.18):
Calcd: C, 74.02; H, 6.17.
Found: C, 73.89; H, 6.14.

Example 27

3,4-dihydro-7-(2-propenoxy)-1[2H]-naphthalenone

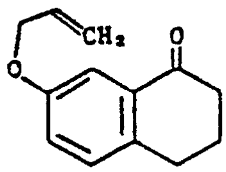

To 22.8g (0.14 mole) of the titled material from Example 26 dissolved in 250ml of methyl ethyl ketone containing 25g (.2M excess) of allyl bromide was added anhydrous $K_2CO_3$ with vigorous stirring. The reaction mixture was refluxed under a $N_2$ blanket for 3 days. Upon cooling to room temperature, the reaction mixture was filtered and the filtrate was stripped of solvent leaving a residue. Chromatography of the residue on silica gel, using 10% ethyl acetate/hexane as eluent, yielded 20g of the titled material which was recrystallized from hexane, m.p. 35°-36° C.

Analysis for $C_{13}H_{14}O_2$ (MW = 202.25):
Calcd: c, 77.20; H, 6.98.
Found: C, 77.35; H, 7.15.

## Example 28

3,4-dihydro-7-hydroxy-8-(2-propenyl)-1[2H]-naphthalenone

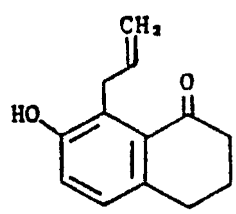

The titled material (10g) from Example 27 was dissolved in 50ml of redistilled diethyl aniline and heated in an oil bath at 200° C for 4 hr. under an Ar blanket. The flask was cooled to room temperature and the residue was diluted with ether and washed with 2N HCl. The organic layer was extracted 2x with 20% NaOH and the basic extracts were acidified with conc. HCl and then extracted with ethyl acetate. The combined organic phases were dried ($Na_2SO_4$) and filtered. The filtrate was evaporated to remove all solvent and the residue was recrystallized from cyclohexane to give 5.38g of the titled material m.p. 99°-101° C. Another 5% can be recovered by chromatography of the mother liquor on silica gel with 1% acetone/toluene as the eluent.

Analysis for $C_{13}H_{14}O_2$ (MW = 202.25):
Calcd: C, 77.20; H, 6.98.
Found: C, 77.30; H, 7.12.

## Example 29

3,4-dihydro-7-hydroxy-8-propyl-1[2H]-naphthalenone

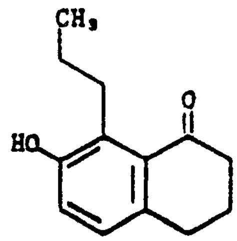

To 3.43g (.017 mole) of the titled material in Example 28 dissolved in 40ml of dry THF in a pressure bottle was added 343mg of 5% Pd/C as catalyst and the contents were hydrogenated at 4 p.s.i at room temperature. After filtration, the filtrate was evaporated down to a residue which was recrystallized from cyclohexane to give 3.4g of the titled material, m.p. 137°-139°C.

Analysis for $C_{13}H_{16}O_2$(MW = 204.27):
Calcd: C, 76.44; H, 7.89.
Found: C, 76.44; H, 7.87.

Example 30

3,4-dihydro-7-(phenylmethoxy)-8-propyl-1[2H]-naphthalenone

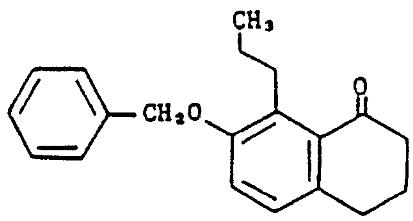

To 100ml of acetone was added 6.0g (.036 mole) of benzylbromide, 10g (.072 mole) of $K_2CO_3$, and 7.12g (.035 mole) of the titled material from Example 29. After refluxing for 24 hr. under $N_2$, the reaction mixture was cooled to room temperature and filtered. The resulting filtrate was evaporated down and the residue placed under vacuum (0.1mm Hg) at 50°C for 2 hr. to remove any excess benzylbromide. The residue was recrystallized from hexane to give 9.25g of the titled material, m.p. 67°-68°C.

Analysis for $C_{20}H_{22}O_2$ (MW = 294.40):
Calcd: C, 80.59; H, 7.53.
Found: C, 80.53; H, 7.60.

Example 31

methyl 1,2,3,4-tetrahydro-7-(phenylmethoxy)-1-oxo-8-propyl-2-naphthalenecarboxylate

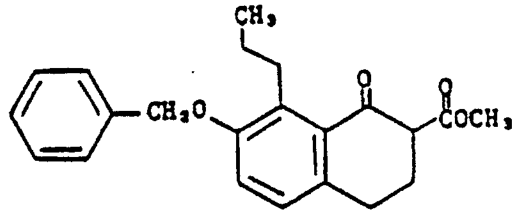

To 100ml of dry THF was added 1.5g (0.06 mole) of NaH, which had been previously washed by hexane, and the system was placed under $N_2$. Dimethyl carbonate, 5.5g (.060 mole) was added and the reaction mixture was warmed to 50° C. While maintaining the system at 50° C, 7.8g (0.026 mole) of the titled material from Example 30 dissolved in 50ml of THF was added dropwise over 30 mm. After addition was complete, the reaction mixture was refluxed for 18 hr. Upon cooling to room temperature, the reaction mixture was acidified with 5ml of acetic acid and diluted with 10 ml of $H_2O$ and 200ml of ether. The organic layer was washed with 0.5N $NaHSO_4$, dried ($Na_2SO_4$), and filtered. Upon removal of the solvent by rotary evaporation, the residue was chromatographed on silica gel and elution by 1% acetone/toluene gave 8.2g of the titled material, which was recrystallized from cyclohexane, m.p. 93°-94° C.

Analysis for $C_{22}H_{24}O_4$ (MW = 352.43):
Calcd: C, 74.98; H, 6.86.
Found: C, 74.73; H, 6.65.

Example 32

ethyl 1,2,3,4-tetrahydro-2-(methoxycarbonyl)-1-oxo-7-(phenylmethoxy)-8-propyl-2-naphthalenepropanoate

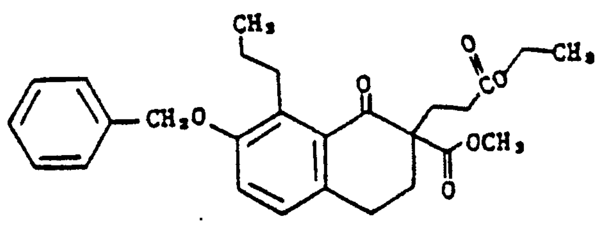

To 8.2g (.023 mole) of the titled material from Example 31 dissolved in 40ml of benzene was added 28mg of hexane washed NaH. Stirring was commenced followed by addition of 2.35g (.023mole) of ethyl acrylate at room temperature over a 15 min. period. After stirring vigorously for 3 days, the reaction was warmed to 70° C for 1 hr. and then cooled to room temperature. Upon addition of 10 ml of water and 50ml of ether to the reaction mixture, the layers were separated and the organic phase dried ($Na_2SO_4$) and filtered. Evaporation of the solvent gave 10g of the crude titled material as an oil.

Analysis for $C_{27}H_{32}O_6$ (MW = 452.53):
Calcd: C. 70.65; H, 7.13.
Found: C. 70.75; H, 7.24.

Example 33

Mixture of methyl 7-hydroxy-1,2,3,4-tetrahydro-1-oxo-8-propyl-2-naphthalenepropanoate

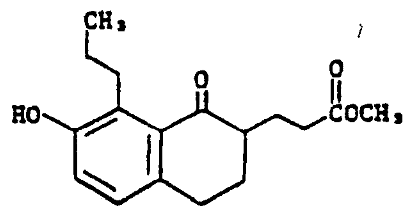

(A)

methyl 7-hydroxy-3,4-dihydro-1-methoxy-8-propyl-2-naphthalenepropanoate

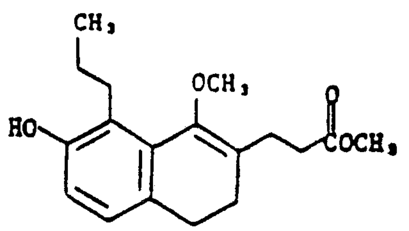

(B)

To 10g (0.022 mole) of the titled material from Example 32 dissolved in 40ml of glacial acetic acid was added 40ml of conc. HCl. After the solution was refluxed for 1 hr. it was cooled and diluted with 75ml of ether. The layers were separated and the organic phase was dried ($Na_2SO_4$) and filtered. Upon evaporation of the solvent, the residue was dissolved in 110ml of methanol containing 20ml of trimethylorthoformate. To this reaction mixture was added 2ml of conc. $H_2SO_4$ and the reaction mixture was stirred for 4 hr. The reaction mixture was poured on ice water and extracted with 150ml of ether. The organic phase was washed with 5% $NaHCO_3$, dried ($Na_2SO_4$) and filtered. Upon removal of the solvent by rotary evaporation, the residue was chromatographed on silica gel, eluting with 5% acetone/toluene, The most non-polar material was found to be "B", the methyl enol ether (2.1g), and the most polar material was found to be compound "A", the tetralone (1.8g).

Compound A

Analysis for $C_{17}H_{22}O_4$ (MW = 290.35):
Calcd: C, 70.31; H, 7.63.
Found: C, 69.82; H, 7.52.

Compound B

Analysis for $C_{18}H_{24}O_4$ (MW = 304.37):
Calcd. C, 71.02; H, 7.94.
Found: C, 71.10; H, 7.95.

Example 34

methyl 7-(3-bromopropoxy)-1,2,3,4-tetrahydro-1-oxo-8-propyl-2-naphthalenepropanoate

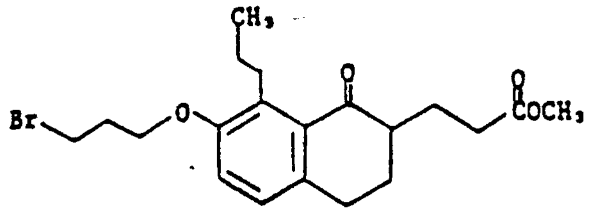

To 25ml of methyl ethyl ketone containing 1.9g (1.5 eq.) of anhydrous potassium carbonate and 18g (.09 mole) of 1,3-dibromopropane was added 1.75g (.006mole) of 2-(3-carbomethoxypropyl)-7-hydroxy-8-n-propyl-1-tetralone from Example 33A. After the reaction mixture was refluxed for 6 days under $N_2$, it was cooled and filtered. The filtrate was evaporated and the residue placed under vacuum (.1mm Hg) at 50°C until no more dibromopropane was evident. Chromatography of the residue on silica gel produced 1.6g of the titled material as an oil when eluted with 1% acetone/toluene.

Analysis for $C_{20}H_{27}O_4Br$ (MW = 411.42):
Calcd: C, 58.39; H, 6.61; Br, 19.44.
Found: C, 58.05; H, 6.42; Br, 19.88.

Example 35

methyl 7-(3-bromopropoxy)-3,4-dihydro-1-methoxy-8-propyl-2-naphthalenepropanoate

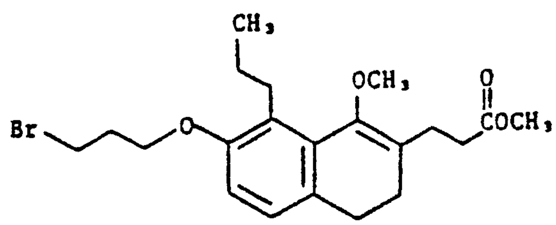

To 30ml of methyl ethyl ketone containing 2g (2.5 eq.) of anhydrous $K_2CO_3$ and 20g (0.1 mole) of 1,3-dibromopropane was added 2g (.0065 mole) of compound B of Example 33. The reaction mixture was refluxed for 7 days and then worked up as in Example 34 to produce 1.43g of the titled material as an oil.

Analysis for $C_{21}H_{29}O_4Br$ (MW = 425.44):
Calcd: C, 59.29; H, 6.87; Br. 18.81.
Found: C, 59.18; H, 6.95; Br. 19.03.

Example 36

methyl 7-[3-(4-acetyl-3-hydroxy-2-propylphenoxy)propoxy]-1,2,3,4-tetrahydro-1-oxo-8-propyl-2-naphthalenepropanoate

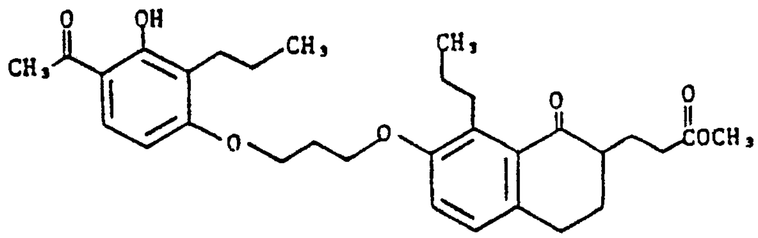

To 15ml of DMF containing 1.5g (3 eq.) of anhydrous $K_2CO_3$ and 775mg (.0039 mole) of 2,4-dihydroxy-3-propylacetophenone was added 1.6g (.0039 mole of the titled material from Example 34. The reaction mixture was stirred overnight under $N_2$ and then filtered. The solvent was removed by rotary evaporation and the residue was redissolved in ethyl acetate and refiltered. Upon removal of the ethyl acetate by evaporation, the residue was chromatographed on silica gel with 2% acetone/toluene as the eluent to give 1.1g of titled material as a solid which was recrystallized from cyclohexane, m.p. = 73°-74° C.

Analysis for $C_{31}H_{40}O_7$ (MW = 524.66):
Calcd: C, 70.97; H, 7.68.
Found: C, 70.51; H, 7.52.

## Example 37

methyl 7-[3-(4-acetyl-3-hydroxy-2-propylphenoxy)propoxy]-3,4-dihydro-1-methoxy-8-propyl-2-naphthalenepropanoate

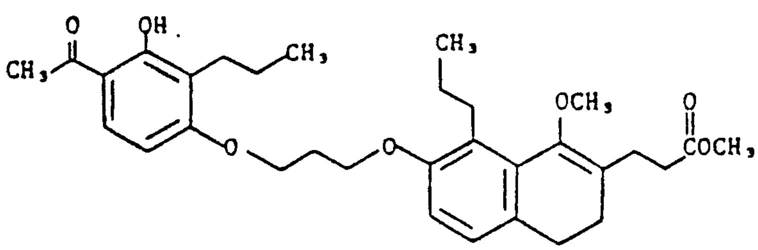

To 15ml of dimethylformamide (DMF) containing 1.5g (3 eq.) of anhydrous $K_2CO_3$ and 640mg (.0033 mole) of 2,4-dihydro-3-n-propylacetophenone was added 1.4g (.0033 mole) of the titled material from Example 35. The reaction mixture was stirred at R.T. for 3 days under $N_2$ then filtered. Upon removal of the solvent by rotary evaporation, the residue was chromatographed on silica gel with elution by 2% acetone/toluene to yield 710mg of the titled material as an oil.

Analysis for $C_{32}H_{42}O_7$ (MW = 538.69):
Calcd: C, 71.34; H, 7.86.
Found: C, 71.32; H, 7.67.

## Example 38

7-[3-(4-acetyl-3-hydroxy-2-propylphenoxy)propoxy]-1,2,3,4-tetrahydro-1-oxo-8-propyl-2-naphthalenepropanoic acid

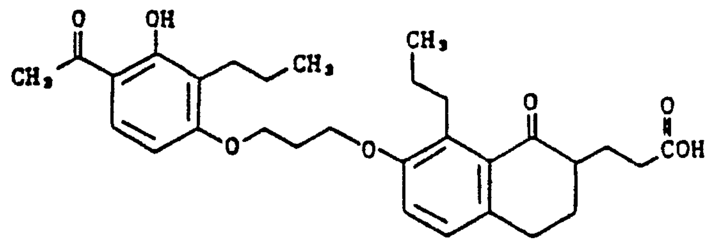

To 10ml of methanol containing 3ml (3 eq.) of 2M LiOH was added 1g (.0019 mole) of the titled material of Example 36 and the reaction mixture was warmed to 40 °C for 3 hr. Afterwards, the reaction was cooled to room temperature and the methanol was removed by evaporation. The aqueous residue was acidified to pH 3.5 with 0.5N $NaHSO_4$. The solid which precipitated out of solution was filtered, air dried and recrystallized from 2B ethanol to give 800mg, m.p. 105 °-107 °C.

Analysis for $C_{30}H_{38}O_7$ (MW = 510.63):
Calcd: C, 70.56; H, 7.50.
Found: C, 70.67; H, 7.65.

## Example 39

7-[3-(4-acetyl-3-hydroxy-2-propylphenoxy)propoxy]-3,4-dihydro-1-methoxy-8-propyl-2-naphthalenepropanoic acid

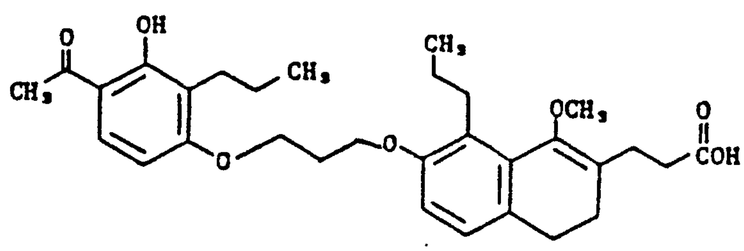

To 7ml of methanol containing 1.5ml (3 eq.) of 2M LiOH was added 650mg (.0012 mole) of the titled material from Example 37. The reaction mixture was warmed to 40 °C for 2 hrs. and then cooled to room temperature. The methanol was removed by rotary evaporation and the aqueous residue was acidified to pH 3.5 with 0.5N $NaHSO_4$. The acidified residue was extracted with ethyl acetate, dried ($Na_2SO_4$) and filtered. The solvent was evaporated but the residue failed to crystallize. The titled product was an oil which weighed 400mg.

Analysis for $C_{31}H_{40}O_7$ (MW = 524.63):
Calcd: C, 70.96: H, 7.68.
Found: C, 70.94: H, 7.56.

## Claims

1. A compound according to the formula:

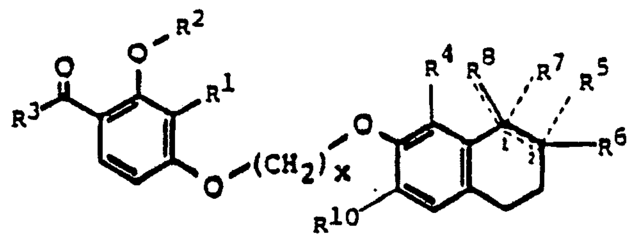

and the pharmaceutically acceptable salts thereof:
wherein $R^1$ represents straight or branched chain alkyl having 2-4 carbon atoms;
wherein $R^2$ represents hydrogen or straight or branched chain alkyl having 1-6 carbon atoms;
wherein $R^3$ represents hydrogen or straight or branched chain alkyl having 1-3 carbon atoms;
wherein $R^4$ and $R^{10}$ are different and represent hydrogen or n-propyl;
wherein X represents an interger from 2 to 5;
wherein $R^5$ is hydrogen, or acts with $R^7$ to produce an additional carbon-carbon bond between $C_1$ and $C_2$;
wherein $R^6$ represents hydrogen or -Z-COOR$^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;
wherein $R^7$ is hydrogen, or acts together with $R^5$ to form an additional carbon-carbon bond between $C_1$ and $C_2$, or acts together with $R^8$ to form

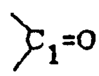

when $R^8$ is oxygen;
wherein $R^8$ is hydrogen, -O-R$^{11}$, or oxygen, such that when R $^8$ is oxygen, $R^8$ acts together with $R^7$ to form

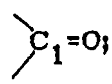

and
wherein $R^{11}$ is straight or branched chain alkyl having 1-4 carbon atoms.

2. A compound according to the formula:

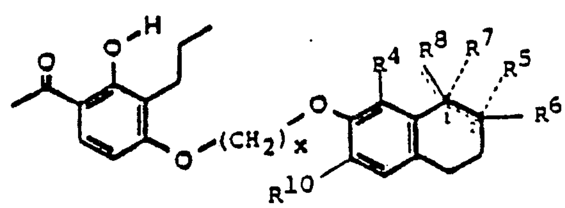

and the pharmaceutically acceptable salts thereof:
wherein X represents an interger from 2 to 5;
wherein $R^4$ and $R^{10}$ are different and represent hydrogen or n-propyl;
wherein $R^5$ represents hydrogen or acts with $R^7$ to produce an additional carbon-carbon bond between $C_1$ and $C_2$;
wherein $R^6$ represents hydrogen -Z-COOR$^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;
wherein $R^7$ is hydrogen, or acts together with $R^5$ to form an additional carbon-carbon bond between $C_1$ and

$C_2$, or acts together with $R^8$ to form

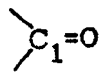

when $R^8$ is oxygen;
wherein $R^8$ is hydrogen, $-O-R^{11}$, or oxygen, such that when $R^8$ is oxygen, $R^8$ acts together with $R^7$ to form

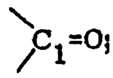

and
wherein $R^{11}$ is straight or branched chain alkyl having 1-4 carbon atoms.

3. A compound according to the formula:

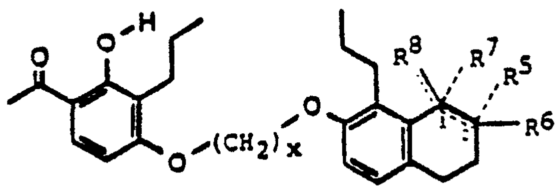

and the pharmaceutically acceptable salts thereof:
wherein $R_5$ represents hydrogen or acts with $R^7$ to produce an additional carbon-carbon bond between $C_1$ and $C_2$;
wherein $R^6$ represents hydrogen or $-Z-COOR^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;
wherein $R^7$ is hydrogen, or acts together with $R^5$ to form an additional carbon-carbon bond between $C_1$ and $C_2$, or acts together with $R^8$ to form

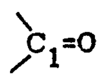

when $R^8$ is oxygen;
wherein $R^8$ is hydrogen, $-O-R^{11}$, or oxygen, such that when $R^8$ is oxygen, $R^8$ acts together with $R^7$ to form

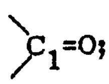

and
wherein $R^{11}$ is straight or branched chain alkyl having 1-4 carbon atoms.

4. A compound according to Claim 2 or 3 of the formula:

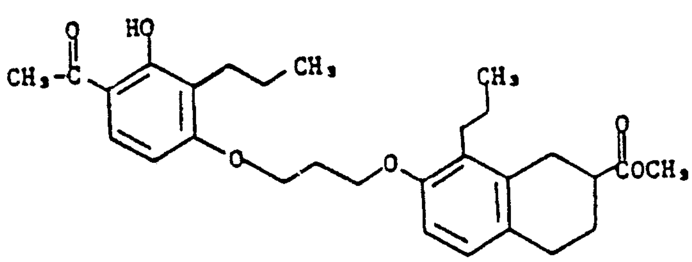

5. A compound according to Claim 2 or 3 of the formula:

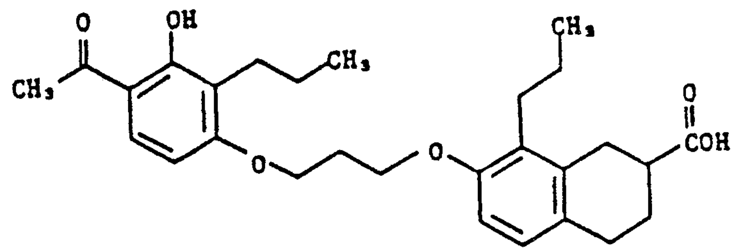

6. A compound according to Claim 2 or 3 of the formula:

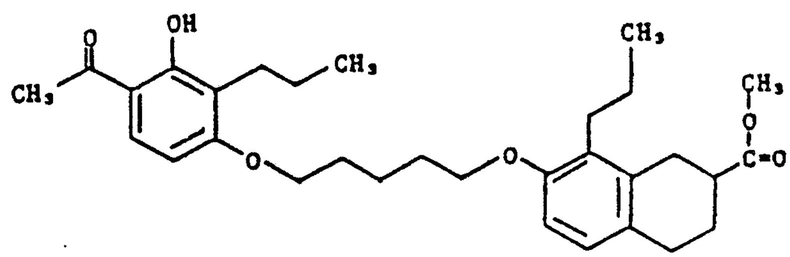

7. A compound according to Claim 2 or 3 of the formula:

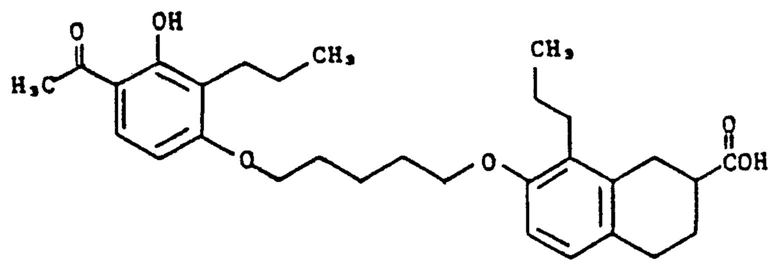

8. A compound according to Claim 2 of the formula:

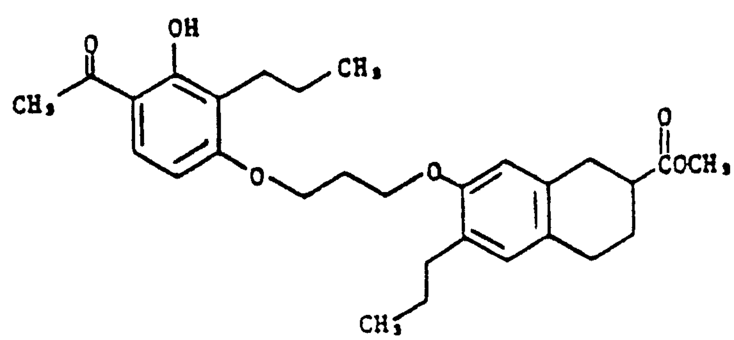

9. A compound according to Claim 2 of the formula:

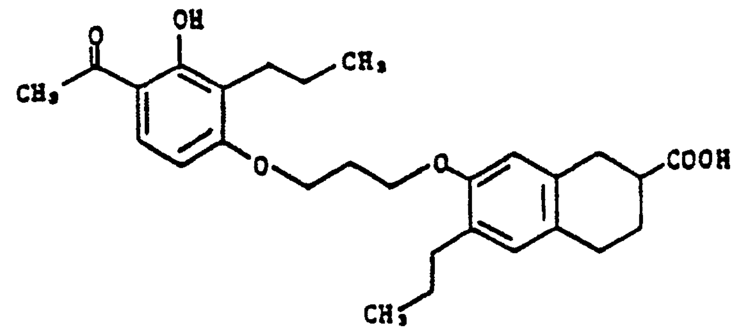

10. A compound according to Claim 2 or 3 of the formula:

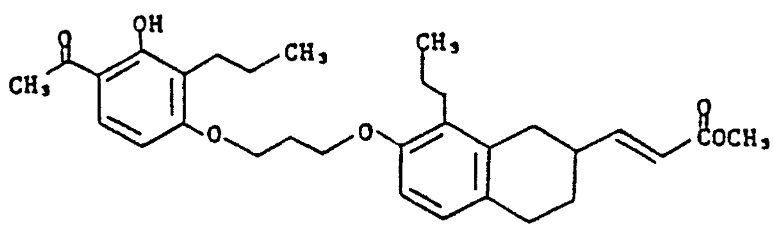

11. A compound according to Claim 2 or 3 of the formula:

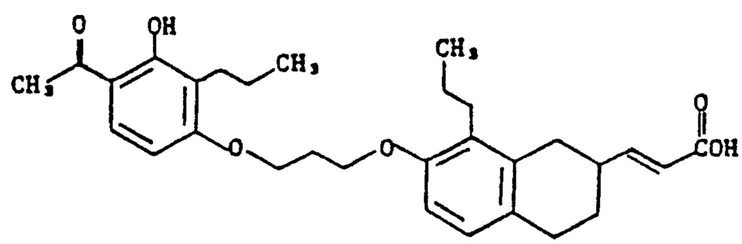

12. A compound according to Claim 2 or 3 of the formula:

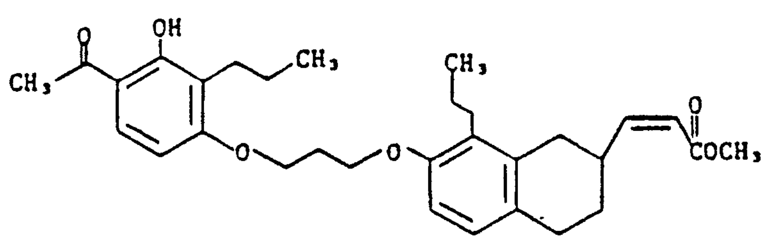

13. A compound according to Claim 2 or 3 of the formula:

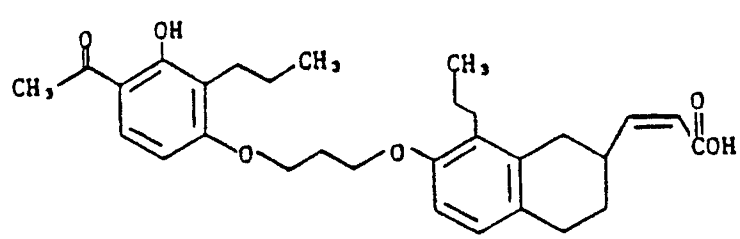

14. A compound according to Claim 2 or 3 of the formula:

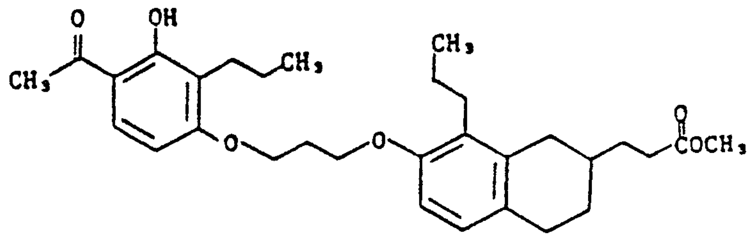

15. A compound according to Claim 2 or 3 of the formula:

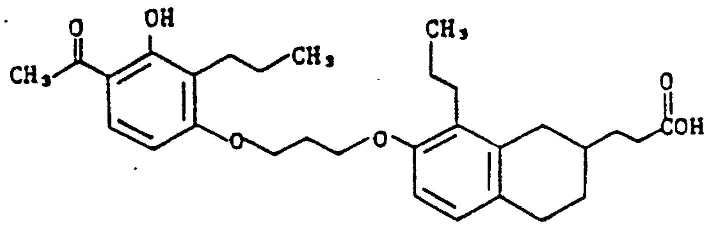

16. A compound according to Claim 2 or 3 of the formula:

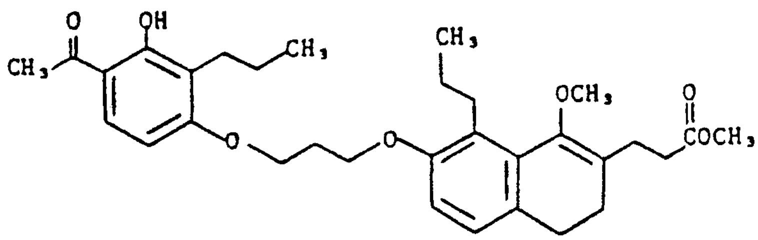

17. A compound according to Claim 2 or 3 of the formula:

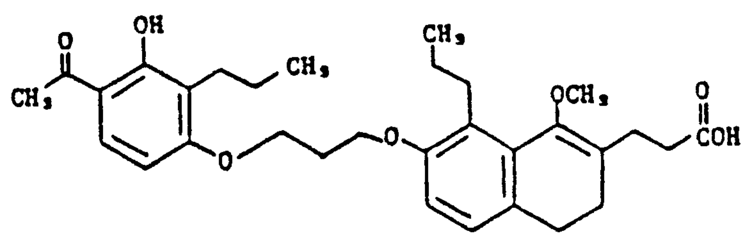

18. A compound according to Claim 2 or 3 of the formula:

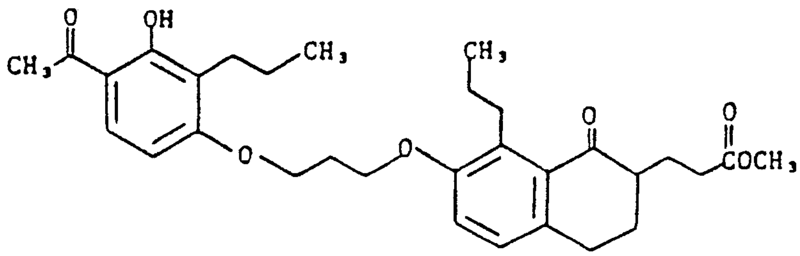

19. A compound according to Claim 2 or 3 of the formula:

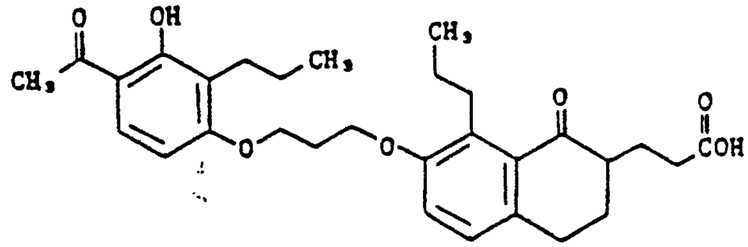

20. A pharmaceutical composition comprising a compound according to Claim 1 and one or more suitable non-toxic carriers.

21. A pharmaceutical composition comprising a compound according to Claim 2 or 3 and one or more suitable non-toxic carriers.

22. Use of a compound according to Claim 1 or 2 for preparing a medicine for treating an inflammatory condition in a mammal.

23. Use of a compound according to Claim 3 for preparing a medicine for treating an inflammatory condition in a mammal.

24. Use of a compound according to Claim 1 or 2 for preparing a medicine for treating an allergic reaction in a mammal.

25. Use of a compound according to Claim 3 for preparing a medicine for treating an allergic reaction in a mammal.

Claims for the following contracting State: GR

1. A compound according to the formula:

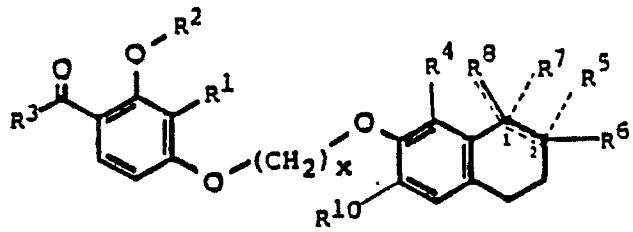

and the pharmaceutically acceptable salts thereof:
wherein $R^1$ represents straight or branched chain alkyl having 2-4 carbon atoms;
wherein $R^2$ represents hydrogen or straight or branched chain alkyl having 1-6 carbon atoms;
wherein $R^3$ represents hydrogen or straight or branched chain alkyl having 1-3 carbon atoms;
wherein $R^4$ and $R^{10}$ are different and represent hydrogen or n-propyl;
wherein X represents an interger from 2 to 5;
wherein $R^5$ is hydrogen, or acts with $R^7$ to produce an additional carbon-carbon bond between $C_1$ and $C_2$;
wherein $R^6$ represents hydrogen or -Z-COOR$^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;
wherein $R^7$ is hydrogen, or acts together with $R^5$ to form an additional carbon-carbon bond between $C_1$ and $C_2$, or acts together with $R^8$ to form

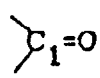

when $R^8$ is oxygen;
wherein $R^8$ is hydrogen, -O-$R^{11}$, or oxygen, such that when $R^8$ is oxygen, $R^8$ acts together with $R^7$ to form

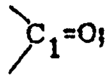

and
wherein $R^{11}$ is straight or branched chain alkyl having 1-4 carbon atoms.

2. A compound according to the formula:

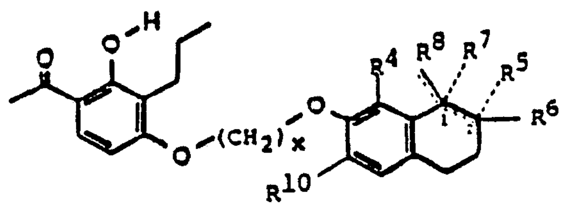

and the pharmaceutically acceptable salts thereof:
wherein X represents an interger from 2 to 5;
wherein $R^4$ and $R^{10}$ are different and represent hydrogen or n-propyl;
wherein $R^5$ represents hydrogen or acts with $R^7$ to produce an additional carbon-carbon bond between $C_1$ and $C_2$;
wherein $R^6$ represents hydrogen -Z-COOR$^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;
wherein $R^7$ is hydrogen, or acts together with $R^5$ to form an additional carbon-carbon bond between $C_1$ and $C_2$, or acts together with $R^8$ to form

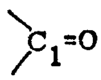

when $R^8$ is oxygen;
wherein $R^8$ is hydrogen, -O-$R^{11}$, or oxygen, such that when R $^8$ is oxygen, $R^8$ acts together with $R^7$ to form

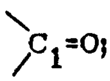

and
wherein $R^{11}$ is straight or branched chain alkyl having 1-4 carbon atoms.

3. A compound according to the formula:

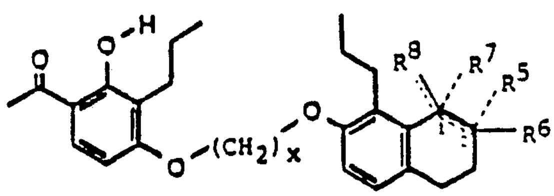

and the pharmaceutically acceptable salts thereof:
wherein $R_5$ represents hydrogen or acts with $R^7$ to produce an additional carbon-carbon bond between $C_1$ and $C_2$;
wherein $R^6$ represents hydrogen or -Z-COOR$^9$, wherein Z is either absent or represents straight or branched chain alkyl or alkenyl having 1-5 carbon atoms; and $R^9$ represents hydrogen, pharmaceutically acceptable cations, or straight or branched chain alkyl having 1-6 carbon atoms;
wherein $R^7$ is hydrogen, or acts together with $R^5$ to form an additional carbon-carbon bond between $C_1$ and $C_2$, or acts together with $R^8$ to form

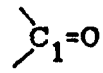

when $R^8$ is oxygen;
wherein $R^8$ is hydrogen, -O-$R^{11}$, or oxygen, such that when $R^8$ is oxygen, $R^8$ acts together with $R^7$ to form

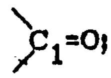

and
wherein $R^{11}$ is straight or branched chain alkyl having 1-4 carbon atoms.

4. A compound according to Claim 2 or 3 of the formula:

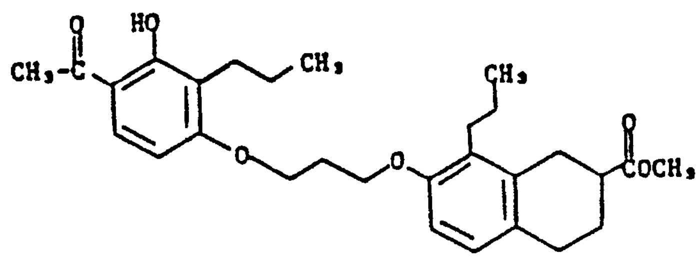

5. A compound according to Claim 2 or 3 of the formula:

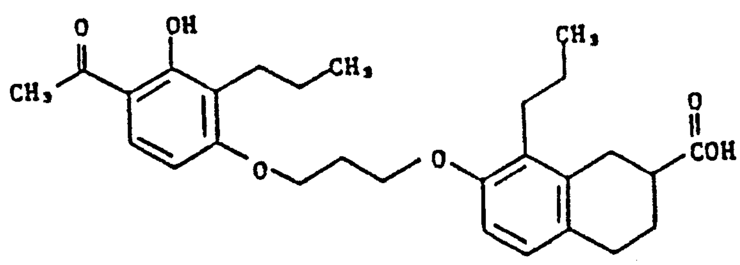

6. A compound according to Claim 2 or 3 of the formula:

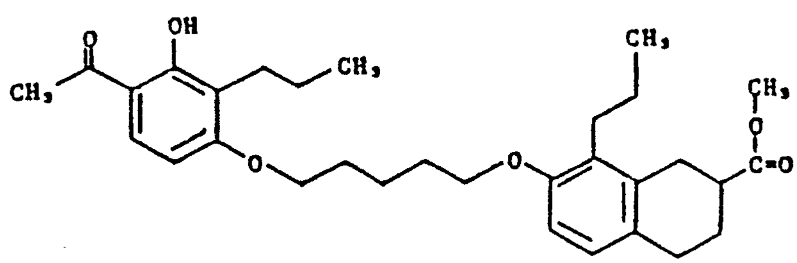

7 A compound according to Claim 2 or 3 of the formula:

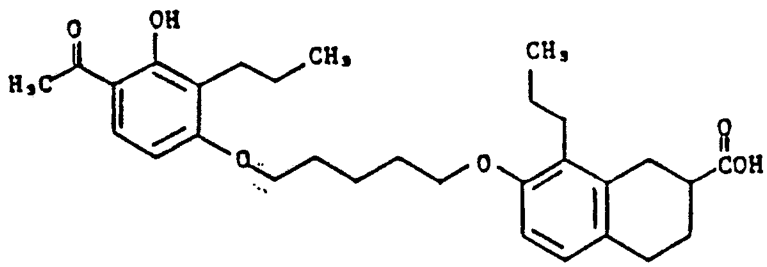

8. A compound according to Claim 2 of the formula:

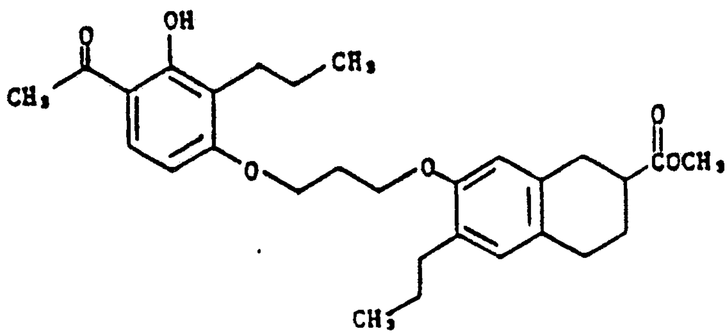

9. A compound according to Claim 2 of the formula:

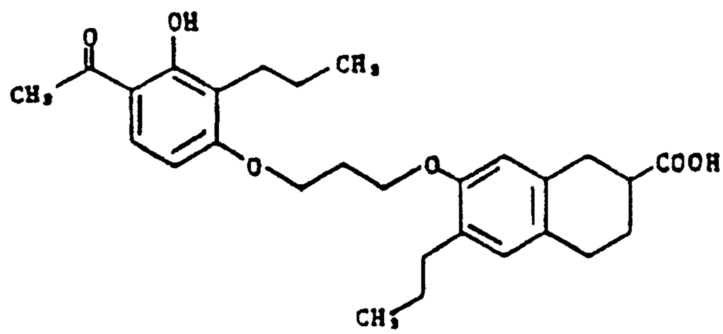

10. A compound according to Claim 2 or 3 of the formula:

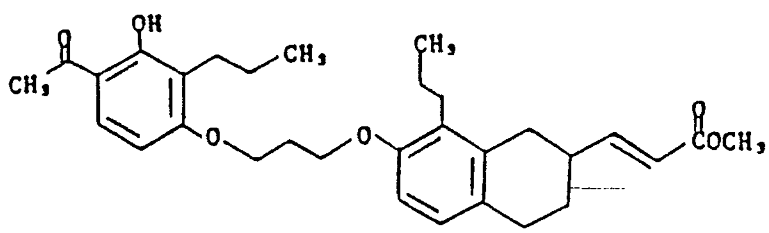

11. A compound according to Claim 2 or 3 of the formula:

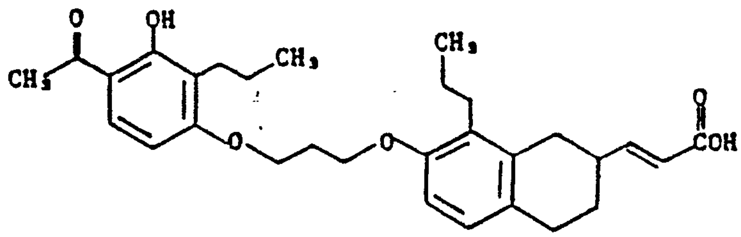

12. A compound according to Claim 2 or 3 of the formula:

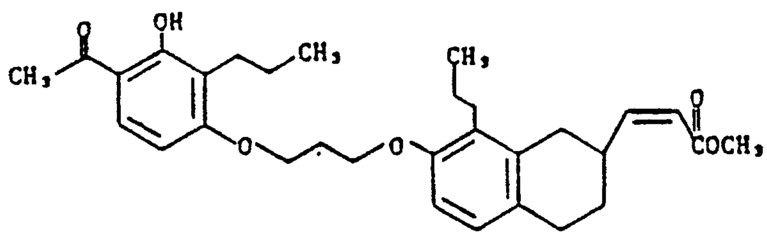

13. A compound according to Claim 2 or 3 of the formula:

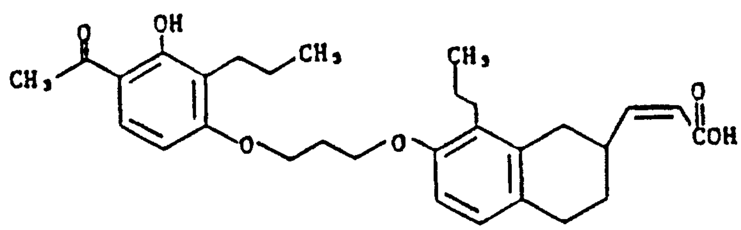

14. A compound according to Claim 2 or 3 of the formula:

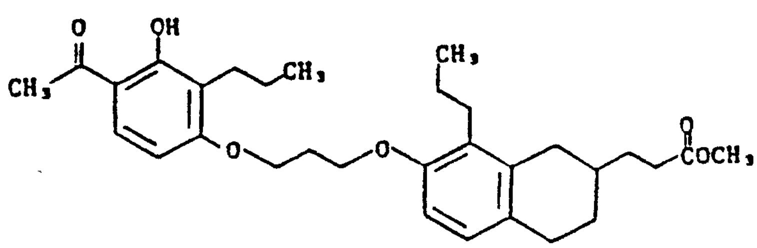

15. A compound according to Claim 2 or 3 of the formula:

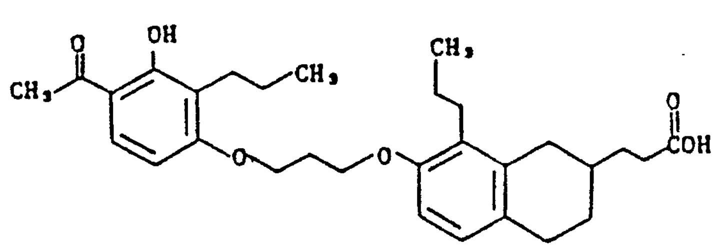

16. A compound according to Claim 2 or 3 of the formula:

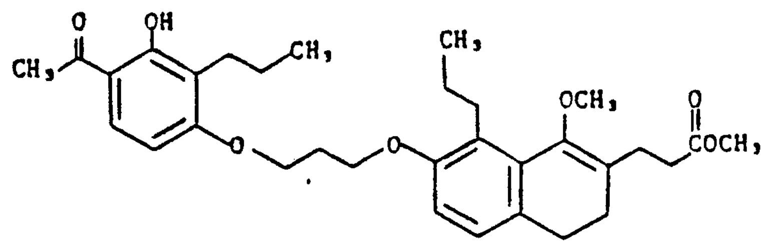

17. A compound according to Claim 2 or 3 of the formula:

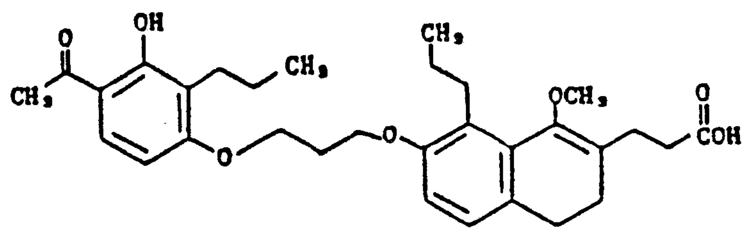

18. A compound according to Claim 2 or 3 of the formula:

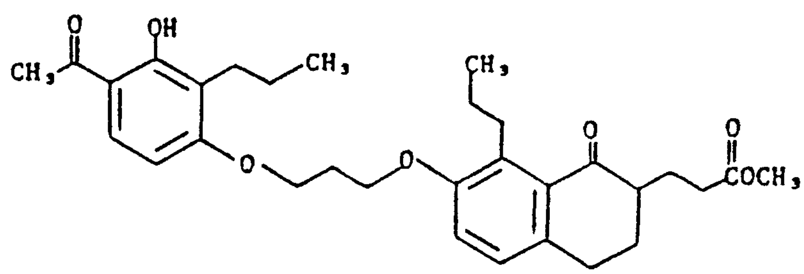

19. A compound according to Claim 2 or 3 of the formula:

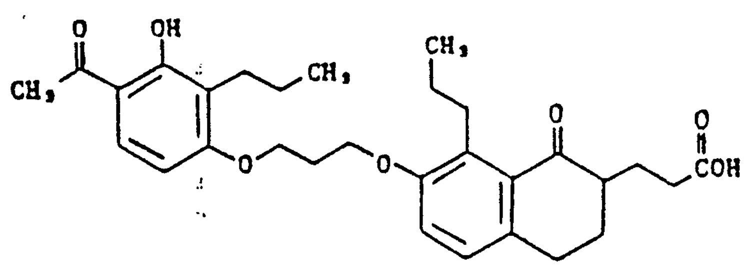

20. Use of a compound according to Claim 1 or 2 for preparing a medicine for treating an inflammatory condition in a mammal.

21. Use of a compound according to Claim 3 for preparing a medicine for treating an inflammatory condition in a mammal.

22. Use of a compound according to Claim 1 or 2 for preparing a medicine for treating an allergic reaction in a mammal.

23. Use of a compound according to Claim 3 for preparing a medicine for treating an allergic reaction in a mammal.